(19) Europäisches Patentamt
European Patent Office
Office européen des brevets

(11) EP 1 394 158 B1

(12) **EUROPEAN PATENT SPECIFICATION**

(45) Date of publication and mention of the grant of the patent:
**21.03.2007 Bulletin 2007/12**

(51) Int Cl.:
***C07D 333/06*** *(2006.01)* ***G03G 5/06*** *(2006.01)*

(21) Application number: **03019529.1**

(22) Date of filing: **29.08.2003**

(54) **Thiophene derivatives and optical elements using the same**

Thiophen Derivate und entsprechende optische Elemente

Dérivés de thiophene et des éléments optiques correspondants

(84) Designated Contracting States:
**AT BE BG CH CY CZ DE DK EE ES FI FR GB GR HU IE IT LI LU MC NL PT RO SE SI SK TR**

(30) Priority: **30.08.2002 JP 2002254953**
**04.02.2003 JP 2003026712**

(43) Date of publication of application:
**03.03.2004 Bulletin 2004/10**

(73) Proprietor: **FUJIFILM Corporation**
**Minato-ku**
**Tokyo (JP)**

(72) Inventors:
- **Nishio, Ryo**
  **Minami-Ashigara-shi**
  **Kanagawa (JP)**
- **Nishikawa, Naoyuki**
  **Minami-Ashigara-shi**
  **Kanagawa (JP)**

(74) Representative: **HOFFMANN EITLE**
**Patent- und Rechtsanwälte**
**Arabellastrasse 4**
**81925 München (DE)**

(56) References cited:
**WO-A-99/18099**
**JP-A- 2000 026 451**
**US-A- 4 861 692**
**US-A- 5 968 697**

- **PATENT ABSTRACTS OF JAPAN vol. 013, no. 349 (P-911), 7 August 1989 (1989-08-07) & JP 01 107262 A (FUJI ELECTRIC CO LTD), 25 April 1989 (1989-04-25)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 394 158 B1

## Description

The present invention relates to a novel compound which is useful as an electro-optical material or a non-linear optical material used in the field of opto-electronics and photonics. The invention also relates to various kinds of optical elements such as phase difference plate and electro-optical devices, which utilize non-linear optical effect.

Description of the Related Art

With respect to non-linear optical materials, search for new materials and device production have been made mainly in the realm of inorganic non-linear optical materials. However, in recent years organic non-linear optical materials have attracted attention for their following properties: (1) large optical non-linearity, (2) quick response, (3) high threshold value for optical damages, (4) capability of versatile molecular design, and (5) excellent production adaptability. Development for the secondary non-linear optical effect requires that polarization induced by electric fields should have no centro symmetricity. Accordingly, it is necessary to arrange molecules showing non-linear optical effect or groups having non-linear optical response in such a structure that lacks centro symmetricity in the material.

In order to arrange molecules showing non-linear optical effect or groups having non-linear optical response in the structure that lacks centro symmetricity, it has been generally utilized to introduce the molecules showing the non-linear optical effect or the groups having non-linear optical response into an organic polymer, and to orient the dipoles, for example, by electric fields. This is a method of orienting dipoles of molecules or response groups showing the secondary non-linear optical effect by applying a high voltage at a temperature higher than the glass transition point of a base polymer and then freezing the orientation of dipoles by cooling. SPIE Journal, vol. 1213, page 7 (1990) gives some examples of electro-optical (EO) modulation device prepared by this method. However, such a material thermally causes orientation relaxation over time, deteriorates in electro-optical characteristics, and as a result lacks stability. Thus, it cannot be practically used or applied to a wide range of use. Its solution has been demanded (see Mol. Cryst. Liq. Cryst. Journal. vol. 189, page 3 (1990)). Further, long time stability has not been insured sufficiently and further improvement has been desired in this respect.

Further, Appl. Phys. Lett. Journal, vol. 34, page 423 (1979) discloses an example of using 5CB (4-cyano-4'-pentylbiphenyl) as a rod-shaped liquid crystal compound for a non-linear optical material. The rod-shaped liquid crystal compound such as 5CB has been poor in non-linear optical characteristics at the molecular level when used as a non-linear optical material, and there has been a demand for a novel compound having superior non-linear optical characteristics.

The present invention is to solve the foregoing problems in the related art and attain the following objects. That is, the invention intends to provide (1) a novel compound that is useful as a non-linear optical material comprising an organic material with no or controlled orientation relaxation, (2) a novel compound which is useful as a non-linear optical material that forms second harmonic waves upon orientation in electric fields, and (3) an optical element, a non-linear optical material, and an electro-optical material using a crosslinked or non-crosslinked body of the compound.

The present inventors have found that these objects can be attained by the invention recited below.

A first aspect of the invention provides a compound represented by the following general formula (I):

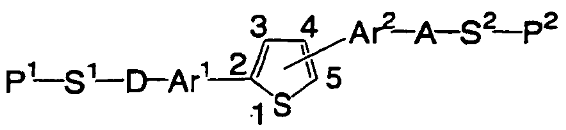

General Formula (I)

In general formula (I), $-Ar^2-A-S^2-P^2$ substitutes on the 4-position or 5-position of a thiophene ring. $Ar^1$ and $Ar^2$ each independently represent a single bond, an aromatic ring having 5 to 14 carbon atoms, or a biphenyl group, provided that at least one of $Ar^1$ and $Ar^2$ represents one of a naphthalene ring and a biphenyl group that may have a substituent. D represents an oxygen atom (-O-), a sulfur atom (-S-), a substituted amino group (-NR-) in which R represents a hydrogen atom, a lower alkyl group having 1 to 6 carbon atoms, or a substituent represented by $P^1-S^1-$, or an amino group having a cyclic structure. A represents an electron attracting group selected from an ester group (-COO-), a sulfonyl group ($-SO_2-$), and a sulfonyloxy group ($-SO_3-$). $S^1$ and $S^2$ each independently represent a bivalent linking group. $P^1$ and $P^2$ each independently represent a substituent consisting of atoms selected from C, H, O, N, S, and a halogen atom, and at least one of $P^1$ and $P^2$ represents a polymerizable group including an acryloyloxy group or a methacryloyloxy group. The thiophene ring may have a substituent.

A second aspect of the invention provides an optical element containing the compound of the first aspect or a

polymer of the compound.

A third aspect of the invention provides a non-linear optical material or an electro-optical material, containing the compound of the first aspect or a polymer formed by polymerizing the compound.

A fourth aspect of the invention provides a compound represented by the following general formula (I-A):

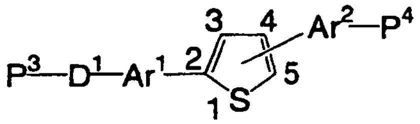

General Formula (I-A)

In general formula (I-A), $-Ar^2-P^4$ substitutes on the 4-position or 5-position of a thiophene ring. $Ar^1$ and $Ar^2$ each independently represent a single bond, an aromatic ring having 5 to 14 carbon atoms, or a biphenyl group, provided that at least one of $Ar^1$ and $Ar^2$ represents one of a naphthalene ring and a biphenyl group that may have a substituent. $P^3$ represents a linear, branched, or cyclic alkyl group having 1 to 20 carbon atoms, wherein one or more of $-CH_2-$ in the alkyl group may be replaced with -O-, -CO-, $-NR^1-$ in which $R^1$ represents a linear, branched, or cyclic alkyl group having 1 to 20 carbon atoms, -CH=CH-, or -C=C-, provided that hetero atoms are not located adjacent to each other, and the alkyl group may have a substituent consisting of atoms selected from C, H, O, N, S, and a halogen atom. $P^4$ represents- $P^{4'}-P^6$, wherein: $P^6$ represents a linear, branched, or cyclic alkyl group having 1 to 20 carbon atoms wherein one or more of-$CH_2$- in the alkyl group may be replaced with -O-, -CO-, -CH=CH-, or -C≡C-, provided that hetero atoms are not located adjacent to each other, and the alkyl group may have a substituent consisting of atoms selected from C, H, O, N, S, and a halogen atom; and $P^{4'}$ represents a bivalent electron attracting group selected from the group consisting of an ester group (-COO-), a sulfonyl group ($-SO_2-$), and a sulfoxy group ($-SO_3-$). $D^1$ represents one selected from the group consisting of an oxygen atom (-O-), a sulfur atom (-S-), and $-NP^5-$. Here, $P^5$ represents a hydrogen atom or a linear, branched, or cyclic alkyl group having 1 to 20 carbon atoms, wherein one or more of $-CH_2-$ in the alkyl group may be replaced with -O-, -CO-,-CH=CH-, or -C≡C-, provided that hetero atoms are not located adjacent to each other, and the alkyl group may have a substituent consisting of atoms selected from C, H, O, N, S, and a halogen atom. $P^5$ may be independent of $P^3$ or bonded with $P^3$ to form a ring. The thiophene ring may have a substituent.

A fifth aspect of the invention provides an optical element containing the compound of the fourth aspect or a polymer thereof.

A sixth aspect of the invention provides a non-linear optical material or an electro-optical material, containing the compound of the fourth aspect or a polymer formed by polymerizing the compound.

Fig. 1 is a view showing a first example of a layer structure of a non-linear optical material in the present invention.
Fig. 2 is a view showing a second example of a layer structure of a non-linear optical material in the present invention.
Fig. 3 is a view showing the change of intensity of second harmonic waves (SH waves) before and after corona poling of compound 2 (maker-fringe view).
Fig. 4 is a view comparing the intensity of the second harmonic waves (SH waves) between compound 23 and 5CB (maker-fringe view).

The present invention will be described in detail below.

The novel compound of the present invention is represented by the following general formula (I):

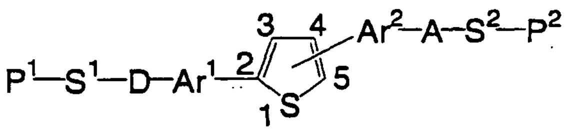

General Formula (I)

In general formula (I), $Ar^1$ and $Ar^2$ each independently represent a single bond, an aromatic ring having 5 to 14 carbon atoms, or a biphenyl group, provided that at least one of $Ar^1$ and $Ar^2$ represents one of a naphthalene ring and a biphenyl group that may have a substituent. D represents an oxygen atom (-O-), a sulfur atom (-S-), a substituted amino group (-NR-) in which R represents a hydrogen atom, a lower alkyl group having 1 to 6 carbon atoms, or a substituent represented by $P^1-S^1-$, or an amino group having a cyclic structure. A represents an electron attracting group

selected from an ester group (-COO-), a sulfonyl group ($-SO_2-$), and a sulfonyloxy group ($-SO_3-$). $S^1$ and $S^2$ each independently represent a bivalent linking group. $P^1$ and $P^2$ each independently represent a substituent consisting of atoms selected from C, H, O, N, S, and a halogen atom, and at least one of $P^1$ and $P^2$ represents a polymerizable group. The thiophene ring may have a substituent.

$Ar^1$ or $Ar^2$ in general formula (I) is preferably an aromatic ring having 5 to 14 carbon atoms (a ring having aromaticity including a hetero ring having 5 or more carbon atoms) or a biphenyl group, and more preferably a benzene ring, a naphthalene ring or a biphenyl group. It is particularly preferred that one of $Ar^1$ and $Ar^2$ is a naphthalene ring. Specifically, it is particularly preferred that $Ar^1$ is a benzene ring and $Ar^2$ is a naphthalene ring, or $Ar^1$ is a naphthalene ring and $Ar^2$ is a benzene ring. When $Ar^1$ or $Ar^2$ represents an aromatic ring having a substituent, examples of preferred substituent include lower alkyl groups having 1 to 6 carbon atoms and, more preferably, methyl group, ethyl group, propyl group, isopropyl group, n-butyl group, sec-butyl group, tert-butyl group, pentyl group and hexyl group.

D in general formula (I) is an oxygen atom (-O-), a sulfur atom (-S-), -NH- as an electron donating group, a substituted amino group (-NR-) in which R is preferably a substituent of a lower alkyl group having 1 to 6 carbon atoms, or a substituent represented by $P^1-S^1-$, and R is particularly preferably a methyl group, an ethyl group, a propyl group, an isopropyl group, a butyl group, a sec-butyl group, a tert-butyl group, a pentyl, a hexyl group, or the like, or an amino group having one of the following cyclic structures:

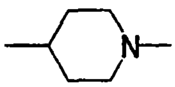
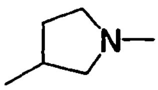
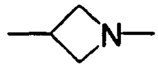

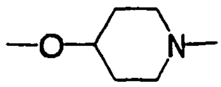
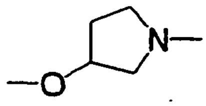
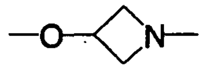

D is particularly preferably an oxygen atom.

A represents an electron attracting group selected from an ester group (-COO-), a sulfonyl group ($-SO_2-$), and a sulfonyloxy group ($-SO_3-$). A is preferably an ester group (-COO-) or a sulfonyloxy group ($-SO_3-$).

The linking groups represented by $S^1$ and $S^2$ in general formula (I) have preferably 2 to 12 carbon atoms, and more preferably 4 to 8 carbon atoms. Further, $S^1$ and $S^2$ may also have a cyclic structure and a cyclohexyl ring is preferred when having the cyclic structure. Examples of linking groups include an alkylene group. The linking group may have one or plural substituents. In this case, examples of the substituents include a substituent having 1 to 4 carbon atoms and a halogen atom. Particularly preferred are a methyl group, an ethyl group, a propyl group and an isopropyl group. Further, when the carbon atom having the substituent is an asymmetric carbon, the steric arrangement thereof may be R, S, or a mixture thereof at an arbitrary rate.

$P^1$ and $P^2$ in general formula (I) each independently represent a substituent consisting of atoms selected from C, H, O, N, S, and a halogen atom. At least one of $P^1$ and $P^2$ represents a polymerizable group including an acryloyloxy group or a methacryloyloxy group. Only one of $P^1$ and $P^2$ may be a polymerizable group but it is preferred that both $P^1$ and $P^2$ are polymerizable groups.

Further, the novel compound of the present invention may be represented by the following general formula (I-A):

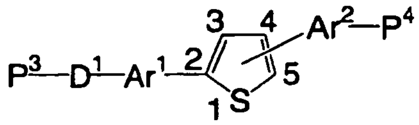

General Formula (I-A)

In general formula (I-A), $Ar^1$ and $Ar^2$ have the same meaning as descried in general formula (I). The thiophene ring may have a substituent.

$D^1$ in general formula (I-A) represents -O-, -S-, $-NP^5-$ in which $P^5$ represents a hydrogen atom or a linear, branched or cyclic alkyl group having 1 to 20 carbon atoms wherein one or more of $-CH_2-$ in the alkyl group may be replaced with -O-, -CO-, -CH=CH- or -C≡C-, provided that hetero atoms are not located adjacent to each other, and may

have a substituent consisting of atoms selected from C, H, O, N, S, and a halogen atom, and $P^5$ may be independent of $P^3$ or bonded with $P^3$ to form a ring. Preferred examples of $D^1$ include -O-, -$NP^3$-, -NR- in which R is a hydrogen atom, a methyl group, an ethyl group, a propyl group, an isopropyl group, a butyl group, a pentyl group, or a hexyl group, or an amino group having one of the following cyclic structures:

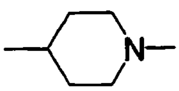
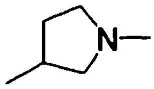
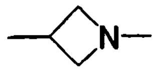

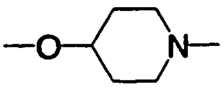
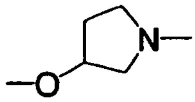
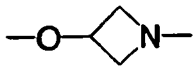

$D^1$ is particularly preferably -O-, -$NP^1$-, or -NR- in which R represents a methyl group, an ethyl group, a propyl group, or an isopropyl group.

$P^3$ in general formula (I-A) represents a linear, branched, or cyclic alkyl group having 1 to 20 carbon atoms. One or more -$CH_2$- in the alkyl group may be replaced with -O-, -CO-, -$NR^1$- in which $R^1$ represents a linear, branched, or cyclic alkyl group having 1 to 20 carbon atoms,-CH=CH-, or -C≡C-, provided that hetero atoms are not located adjacent to each other. $P^3$ may have a substituent consisting of atoms selected from C, H, O, N, S, and a halogen atom. Examples of $P^3$ include a linear, branched or cyclic alkyl group wherein one or more hydrogen atom(s) at an arbitrary position may also be substituted with a halogen atom, a polyethyleneoxy group, or the following polymerizable substituent (q is an integer):

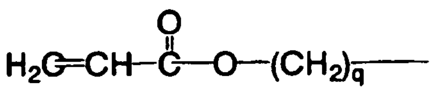
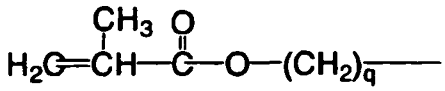

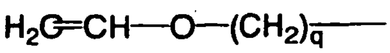
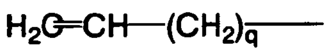

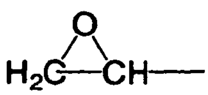

q is preferably 2 to 14, more preferably 2 to 10, and still more preferably 4 to 10. $P^3$ is particularly preferably a linear or branched alkyl group having 1 to 14 carbon atoms, and further preferably a linear alkyl group having 1 to 12 carbon atoms.

Examples of $P^4$ include the following substituent groups:

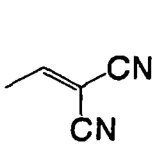
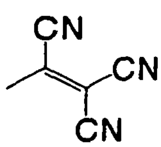

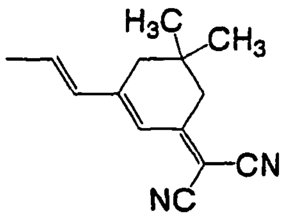 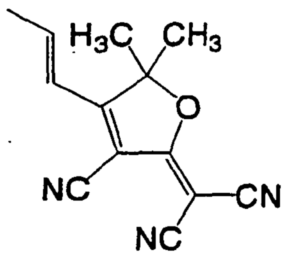

$-P^4$ in general formula (I-A) represents $-P^{4'}-P^6$, $P^6$ represents a linear, branched, or cyclic alkyl group having 1 to 20 carbon atoms wherein one or more $-CH_2-$ in the alkyl group may be replaced with -O-,-CO-, -CH=CH-, or -C≡C-, provided that hetero atoms are not located adjacent to each other, and the alkyl group may have a substituent consisting of atoms selected from C, H, O, N, S, and a halogen atom, and $-P^{4'}-$ represents a bivalent electron attracting group consisting of atoms selected from the group consisting of an ester group (-COO-), a sulfonyl group ($-SO_2-$), and a sulfoxy group ($-SO_3-$).

Examples of $P^6$ include a linear, branched or cyclic alkyl group (one or more hydrogen atom(s) at an arbitrary position may be substituted with a halogen atom), a polyethyleneoxy group, and the following polymerizable substituent groups (q is an integer):

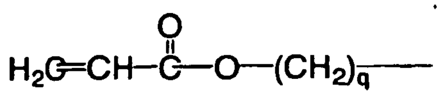 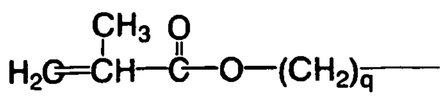

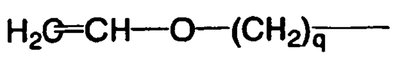 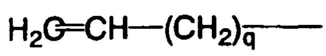

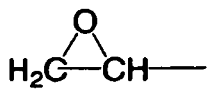

q is preferably 2 to 14, more preferably 2 to 10, and further preferably 4 to 10.

Examples of $P^{4'}$ include an ester group (-COO-), sulfonyl group ($-SO_2-$), a sulfoxy group ($-SO_3-$), or the following substituents:

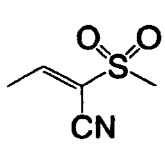 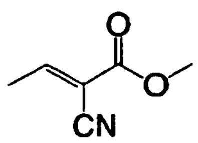 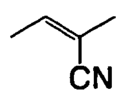

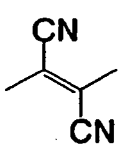 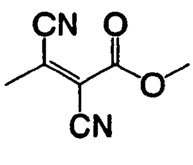 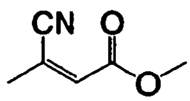

$-Ar^2-A-S^2-P^2$ in general formula (I) and $-Ar^2-P^4$ in general formula (I-A) substitute on the 4-position or 5-position of the thiophene ring and they preferably substitute on the 5-position in the invention and, specifically, the compounds represented by general formulae (I) and (I-A) are preferably the compounds represents by the following general formulae (II) and (II-A).

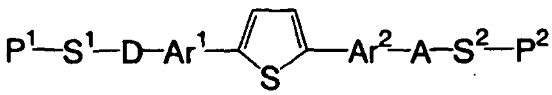

General Formula (II)

In general formula (II), $Ar^1$, $Ar^2$, D, A, $S^1$, $S^2$, $P^1$, and $P^2$ have the same meanings as described above.

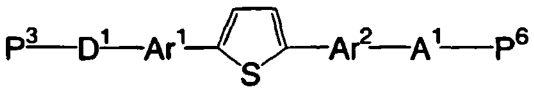

General Formula (II-A)

In general formula (II-A), $Ar^1$, $Ar^2$, D', A', $P^3$, and $P^6$ have the same meanings as in general formula (I-A).

In general formula (I), a compound satisfying a combination of the following constitutions (1) to (4) is preferable:

(1) $Ar^1$ is a benzene ring and $Ar^2$ is a naphthalene ring, or $Ar^1$ is a naphthalene ring and $Ar^2$ is a benzene ring.
(2) $S^1$ and $S^2$ each independently represent an alkylene group having 2 to 12 carbon atoms.
(3) $P^1$ and $P^2$ each independently represent an acryloyloxy group or methacryloyloxy group.
(4) D represents an oxygen atom (-O-), sulfur atom (-S-), substituted amino group (-NR-) in which R represents a hydrogen atom, a lower alkyl group having 1 to 6 carbon atoms, or a substituent represented by $P^1-S^1-$ which may be an amino group having a cyclic structure. A represents an electron attracting group selected from an ester group (-COO-), a sulfonyl group ($-SO_2-$) and sulfonyloxy group ($-SO_3-$).

In general formula (II-A), a compound satisfying a combination of the following constitutions (1) to (3) is most preferable:

(1) $Ar^1$ is a 1,4-phenylene group and $Ar^2$ is a 2,6-naphthalene group, or $Ar^1$ is a 2,6-naphthalene group and $Ar^2$ is a 1,4-phenylene group.
(2) $P^3$ and $P^6$ each independently represent an alkyl group having 1 to 16 carbon atoms.
(3) $D^1$ represents an oxygen atom (-O-), a sulfur atom (-S-), a substituted amino group (-NR-) in which R represents a hydrogen atom, a lower alkyl group having 1 to 6 carbon atoms, or a substituent represented by $P^3$, or an amino group having a cyclic structure. $A^1$ represents an electron attracting group selected from -COO-, $-SO_2-$, or $-SO_3-$.

Examples of the compound represented by general formula (I) include the following compounds.

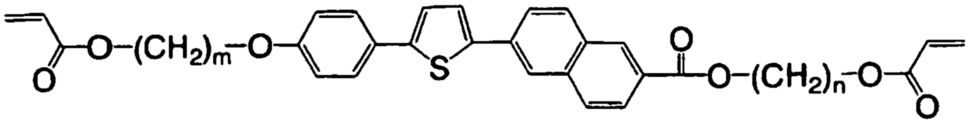

Compound 1 m=2, n=2
Compound 2 m=4, n=4
Compound 3 m=6, n=6
Compound 4 m=12, n=12
Compound 5 m=4, n=6

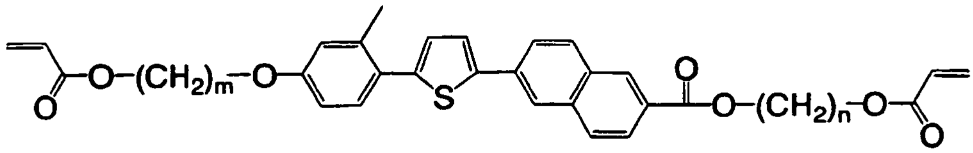

Compound 6 m=4, n=4

Compound 7 m=6, n=6

Compound 8 m=8, n=8

Compound 9 m=10, n=10

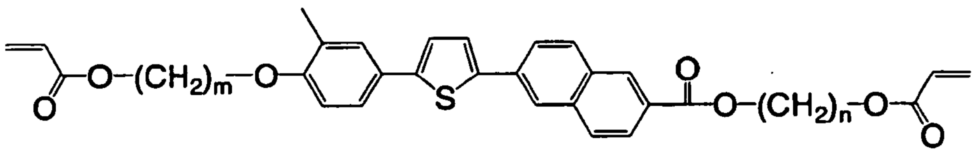

Compound 10 m=6, n=6

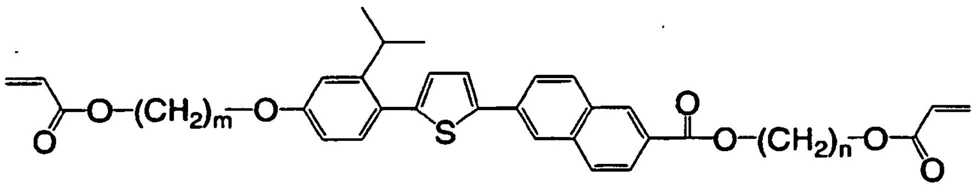

Compound 11 m=6, n=6

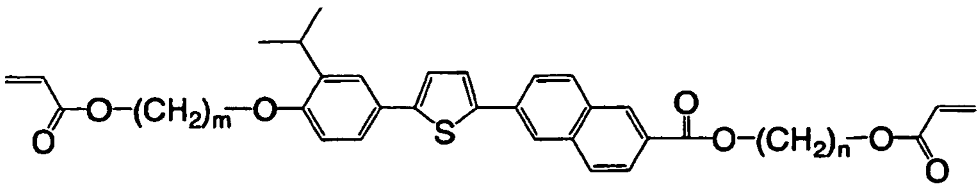

Compound 12 m=6, n=6

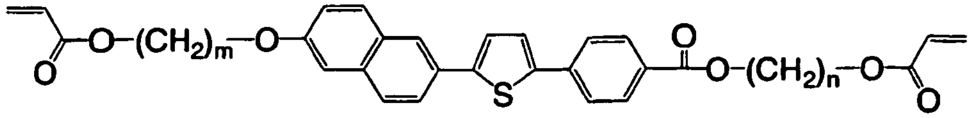

Compound 13 m=6, n=6

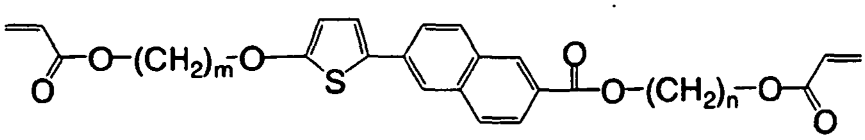

Compound 14 m=6, n=6

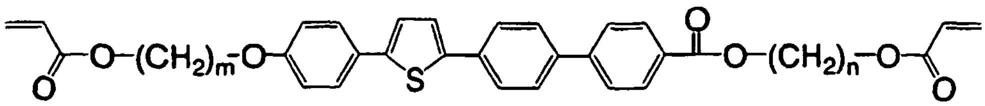

Compound 15 m=6, n=6

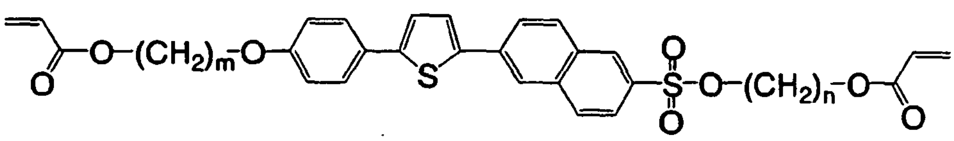

Compound 16 m=6, n=6

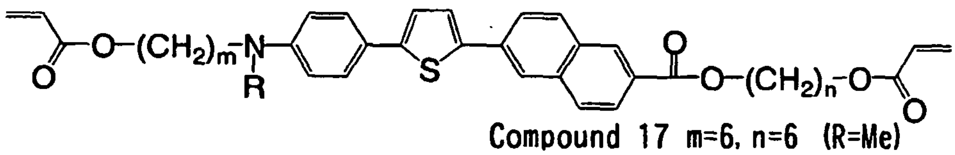

Compound 17 m=6, n=6 (R=Me)
Compound 18 m=6, n=6 (R=i-Pr)

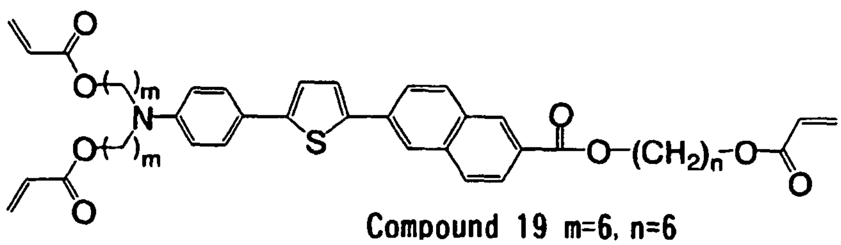

Compound 19 m=6, n=6

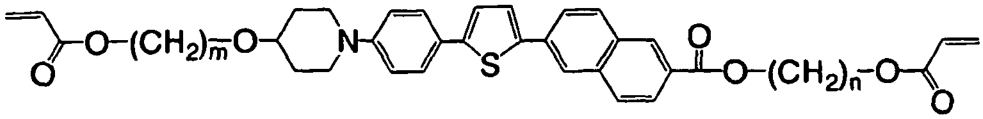

Compound 20 m=6, n=6

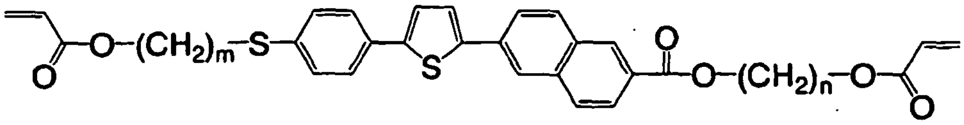

Compound 21 m=6, n=6

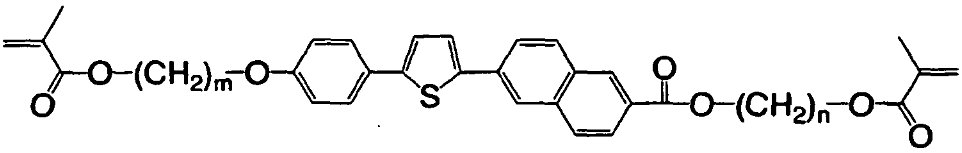

Compound 22 m=6, n=6

Examples of the compound represented by general formula (I-A) include the following compounds.

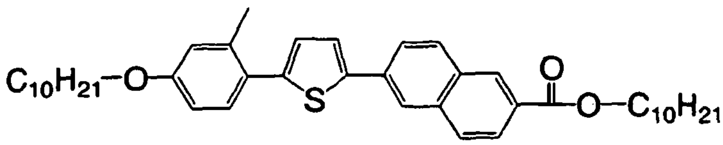

Compound 23

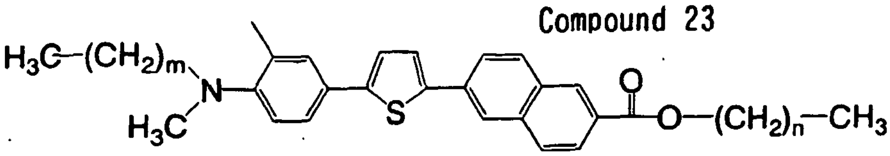

Compound 24 m=7, n=7

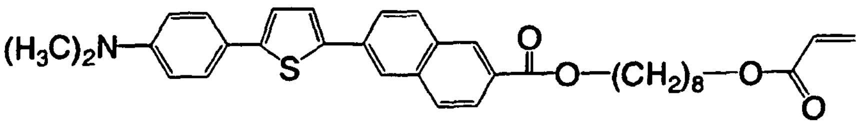

Compound 25

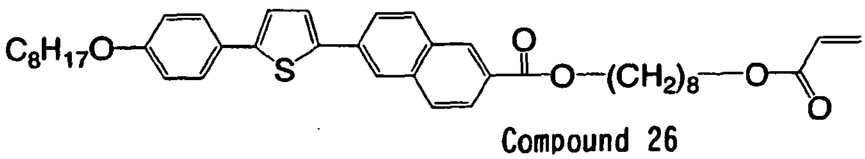

Compound 26

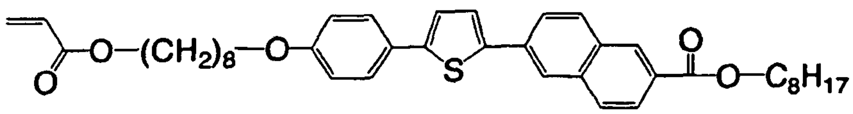

Compound 27

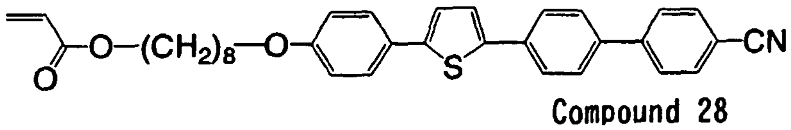

Compound 28

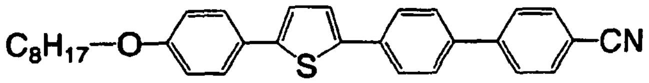

Compound 29

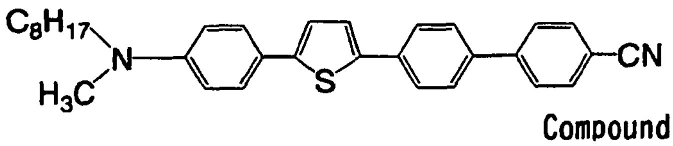

Compound 30

The preferred examples of compounds of the invention may be synthesized by the following method of schemes 1 and 2. However, the present invention is not be limited by these schemes.

Scheme 1

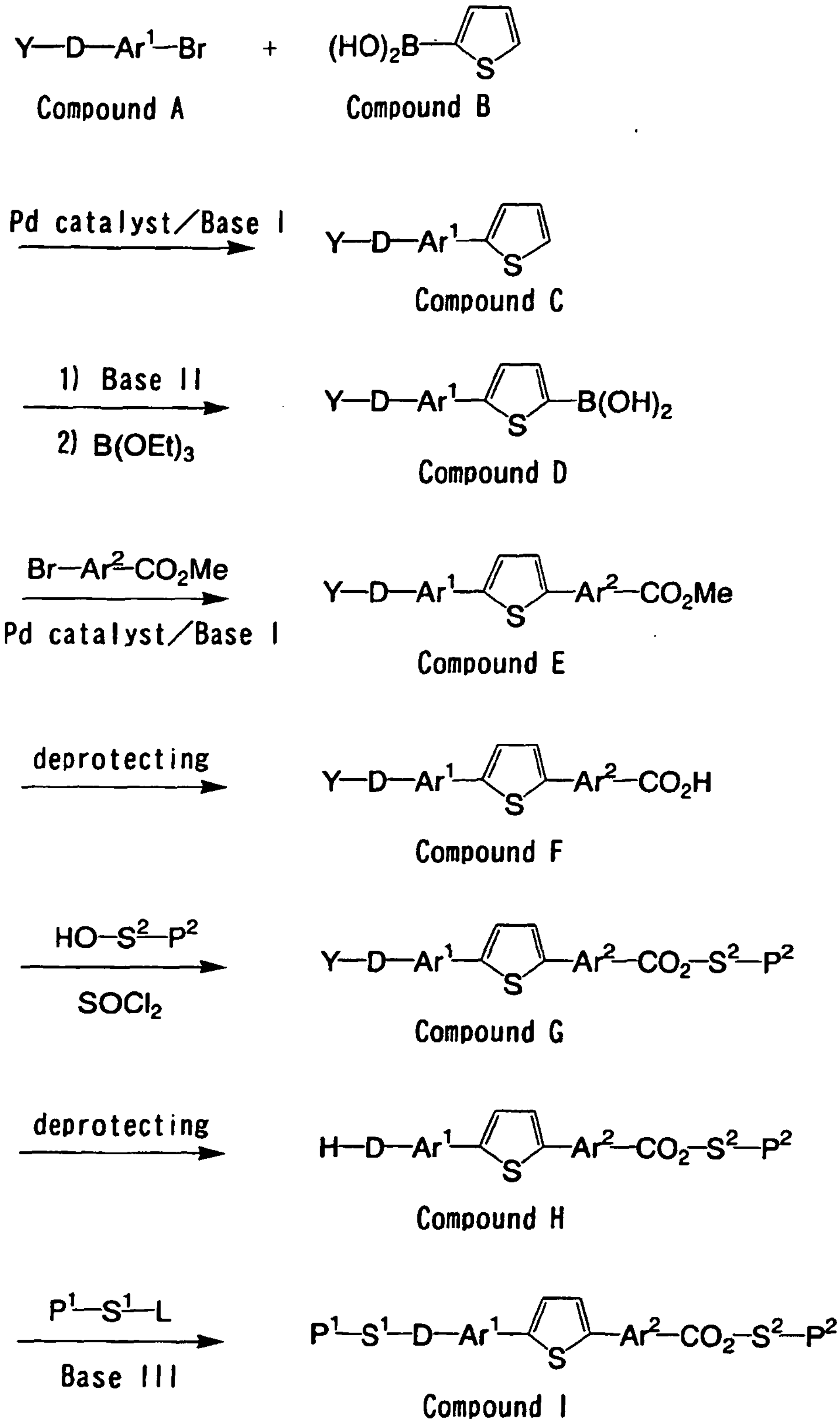


In the scheme 1, $Ar^1$, $Ar^2$, D, $S^1$, $S^2$, $P^1$, and $P^2$ have the same meanings as described above and L represents a leaving group and Y represents a protective group.

In the method of scheme 1, when the compound represented by compounds A and B (the compound B is preferably 1 to 2 equivalent amount and more preferably 1 to 1.3 equivalent amount to the compound A by the equivalent

amount ratio) are used as the starting material and stirred in the presence of a Pd catalyst (Pd catalyst is, preferably, 0.1 to 5 mol% and, further preferably, 1 to 2 mol% based on the compound A) and the base I (base I is preferably 1 to 4 equivalent amount and, more preferably, 1 to 2 equivalent amount to the compound B) in an organic solvent under an overheating condition at about 50 to 150°C for several hours, a compound represented by the compound C can be obtained. Examples of the protective group Y in the compound A include tert-butoxycarbonyl (Boc) group, benzoylcarbonyl (Cbz) group, acetyl (Ac) group or the like, for example, when D is an amino group. When D is other substituent than described above, protecting and deprotecting can be conducted in accordance with the method as described in Protective Groups in Organic Chemistry, Plenum Press (London and New York, 1973); Green, T. W., Protective Groups in Organic Synthesis, Wiley New York, 1981; and Peptides, Vol. I, Schrooder and Lubke, Academic Press (London and New York, 1965).

Examples of the Pd catalyst used in the reaction are preferably, $Pd(PPh_3)_4$, $PdCl_2(PPh_3)_2$, $Pd(OAc)_2$-$PPh_3$, and $Pd_2(dba)_3CHCl_3$-$PPh_3$. For base I, inorganic and organic bases can be used and preferred examples of the base may include potassium carbonate, sodium carbonate, sodium hydroxide, potassium hydroxide, lithium hydroxide, potassium acetate, sodium acetate and sodium phosphate. Examples of the organic solvent to be used include N,N-dimethylformamide (DMF), N,N-dimethylacetoamide (DMA), tetrahydrofuran (THF), chloroform, dichloromethane and dimethylsulfoxide (DMSO).

Then, when the compound C is stirred under the presence of the base II (in which the base II is preferably 1 to 2 equivalent amount, and further preferably 1 to 1.2 equivalent amount to the compound C) at a low temperature of 0°C or lower for several hours and then $B(OEt)_3$ is dropped and stirred at a room temperature for several hours, a boronic acid compound represented by the compound D is obtained. Preferred examples of the base II include tert-butyl lithium, n-butyl lithium, lithium diisopropylamide (LDA), sodium hydride, lithium hydride and potassium carbonate and, particularly preferably, include tert-butyl lithium, n-butyl lithium and lithium diisopropylamide (LDA).

Then, when the compound D and the compound represented by Br-$Ar^2$-$CO_2Me$ are stirred under the presence of the Pd catalyst and the base I, the compound E can be obtained.

Then, when the methyl ester of the compound E is hydrolyzed by the method described in the "Protective Groups in Organic Chemistry", the compound F is obtained.

Then, after adding thionyl chloride to the compound F, when the compound represented by HO-$S^2$-$P^2$ is dropped and stirred for several hours, the compound G is obtained.

Then, when the protective group Y of the compound G is deprotected by the method described in the "Protective Groups in Organic Chemistry", the compound H can be obtained.

Finally, when the compound H is reacted with the compound represented by $P^1$-$S^1$-L under the presence of the base III, the aimed compound I is obtained. Preferred base III may include sodium hydroxide, potassium hydroxide, potassium carbonate, sodium carbonate, and lithium hydroxide. Preferred examples of the leaving group L include halogen atom, alkyl sulfonyloxy group or arylsulfonyloxy group.

Scheme 2 ($S^1$ and $S^2$ are identical, and $P^1$ and $P^2$ are identical)

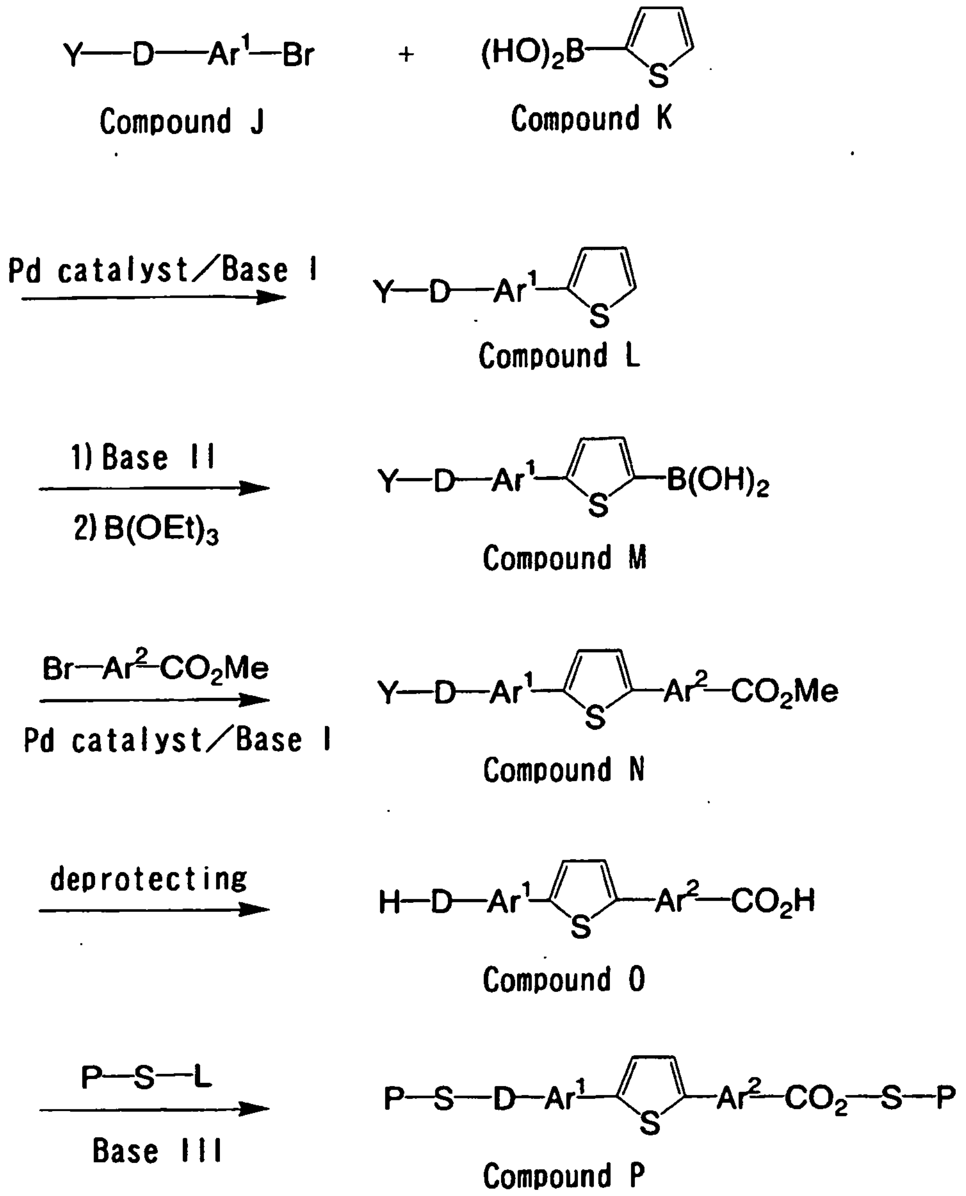


Compound J

Compound K

Compound L

Compound M

Compound N

Compound O

Compound P

In the scheme 2, $Ar^1$, $Ar^2$ and D have the same meanings as described above, L represents a leaving group, Y represents a protective group, S represents a spacer, and P represents a polymerizable group.

In the method of scheme 2, when the compound represented by compounds J and K (compound K is preferably 1 to 2 equivalent amount and, more preferably, 1 to 1.3 equivalent amount to the compound J by the equivalent amount ratio) are used as the starting material and stirred in the presence of a Pd catalyst (Pd catalyst is, preferably, 0.1 to 5 mol% and, further preferably, 1 to 2 mol% based on the compound J) and the base I (base I is, preferably, 1 to 4 equivalent amount and, more preferably, 1 to 2 equivalent amount to the compound B) in an organic solvent under an overheating condition at about 50 to 150°C for several hours, a compound represented by the compound L can be obtained. Examples of the protective group Y in the compound J include tert-butoxycarbonyl (Boc) group, benzoylcarbonyl (Cbz) group, acetyl (Ac) group or the like, for example, when D is an amino group. When D is a substituent other than described above, protecting and deprotecting can be conducted in accordance with the method as described in "Protective Groups in Organic Chemistry Journal".

Examples of the Pd catalyst used in the reaction are preferably, $Pd(PPh_3)_4$, $PdCl_2(PPh_3)_2$, $Pd(OAc)_2$-$PPh_3$, and $Pd_2(dba)_3CHCl_3$-$PPh_3$. For base I, inorganic and organic bases can be used and preferred examples of the base

may include potassium carbonate, sodium carbonate, sodium hydroxide, potassium hydroxide, lithium hydroxide, potassium acetate, sodium acetate and sodium phosphate. Examples of the organic solvent to be used include N,N-dimethylformamide (DMF), N,N-dimethylacetoamide (DMA), tetrahydrofuran (THF), chloroform, dichloromethane and dimethylsulfoxide (DMSO).

Then, when the compound L is stirred under the presence of the base II (in which the base II is, preferably, 1 to 2 equivalent amount, further preferably, 1 to 1.2 equivalent amount to the compound L) at a low temperature of 0°C or lower for several hours and then $B(OEt)_3$ is dropped and stirred at a room temperature for several hours, a boronic acid compound represented by the compound M is obtained. Preferred examples of the base II include tert-butyl lithium, n-butyl lithium, lithium diisopropylamide (LDA), sodium hydride, lithium hydride and potassium carbonate and, particularly preferably, include tert-butyl lithium, n-butyl lithium and lithium diisopropylamide (LDA).

Then, when the compound M and the compound represented by $Br\text{-}Ar^2\text{-}CO_2Me$ are stirred under the presence of the Pd catalyst and the base I, the compound N can be obtained.

Then, when the protective group Y of the compound N is deprotected by the method described in the "Protective Groups in Organic Chemistry", the compound O can be obtained.

Finally, when the compound O is reacted with the compound represented by P-S-L under the presence of the base III, the aimed compound P is obtained. Preferred base III may include sodium hydroxide, potassium hydroxide, potassium carbonate, sodium carbonate and lithium hydroxide. Preferred examples of the leaving group L include halogen atom, alkyl sulfonyloxy group or arylsulfonyloxy group.

The optical element as the application use of the compound represented by general formula (I) of the invention may include functional films (for example, optical films, ferroelectric films, anti-ferroelectric films, and piezoelectric films) and functional devices (for example, non-linear optical devices, electro-optical devices, pyroelectric devices, piezoelectric devices and optical modulation devices) utilized in the optical field and the electronics field. Specifically, in the application use of the non-linear optical devices (electro-optical devices), a wave guide type device is prepared, for example, as described in "Optical Wave Optics", issued from Corona Co., (1998), page 200, which is then utilized as an optical modulator for modulating the phase or intensity of optical waves or optical switch. Further, JP-A No. 9-22035 discloses an example for the use in optical signal generators and JP-A No. 2001-264715 discloses an example of manufacturing electric waves-optical signal converters.

Scheme 3

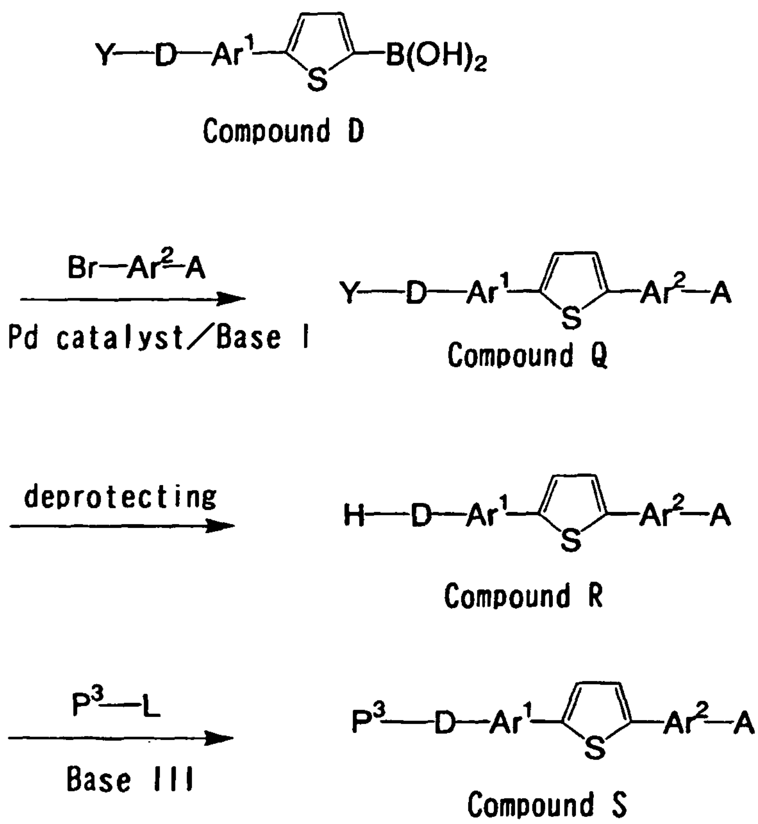


Compound D

Compound Q

Compound R

Compound S

In the scheme 3, $Ar^1$, $Ar^2$, D, A, and $P^3$ have the same meanings as those for scheme 2, L represents a leaving group and Y represents a protective group. The compound D is synthesized in accordance with the method of the scheme 1. Then, when the compound D and the compound represented by Br-$Ar^2$-A are stirred under the presence of the Pd catalyst and the base I, the compound Q is obtained (compound represented by Br-$Ar^2$-A is, preferably, 1 to 2 equivalent amount and, more preferably, 1 to 1.3 equivalent amount to the compound D by equivalent amount ratio). Then, when the protective group Y of the compound Q is deprotected by the method described in "Protective Groups in Organic Chemistry", the compound R is obtained.

Finally, when the compound R is reacted with the compound represented by $P^3$-L under the presence of the base III, the aimed compound S is obtained (compound represented by $P^3$-L is, preferably, 1 to 2 equivalent amount, more preferably, 1 to 1.3 equivalent amount to the compound R by the equivalent amount ratio). Preferred examples of the base III include sodium hydroxide, potassium hydroxide, potassium carbonate, sodium carbonate and lithium hydroxide. Preferred examples of the leaving group L may include halogen atom, alkyl sulfonyloxy group or arylsulfonyloxy group. As the reaction solvent, the organic solvent used in the schemes 1 and 2 can be utilized.

(Optical element)

The examples of an optical element of the invention include functional films (for example, optical films, ferroelectric films, anti-ferroelectric films, piezoelectric films) and functional devices (for example, non-linear optical devices, electro-optical devices, pyroelectric devices, piezoelectric devices and optical modulation devices) utilized in the field in the optical field or electronics field.

(Non-linear optical material and electro-optical material)

The non-linear optical material and the electro-optical material of the invention contain the compound of the invention, as at least one constituent component, or otherwise contain a polymer polymerized from the compound of the invention, as at least one constituent component. When the compound of the invention has a crosslinking group, the polymer polymerized from the compound of the invention may be used as a non-linear optical material and an electro-optical material. The non-linear optical material and the electro-optical material of the invention include two embodiments. One is an embodiment in which the non-linear optical material and the electro-optical material are made of the compound of the invention alone. The other is an embodiment in which the non-linear optical material and the electro-optical material are made of the compound of the invention and a medium. The producing methods thereof will be shown below.

- Producing method using the compound of the invention alone -

The non-linear optical material and the electro-optical material of the invention can be produced by (1) coating or sandwiching a monomer composition containing the compound of the invention to a support or the like; (2) orienting the same; and (3) crosslinking the same while applying an orientation treatment. In this case, the compound of the invention preferably has a crosslinking substituent.

- Producing method using the compound of the invention and a medium -

The material can be produced by (1) dissolving a composition containing the compound of the invention and a polymeric medium supporting the same, or a composition containing a polymer of the compound of the invention into a solvent; (2) coating or sandwiching the same to a support followed by drying; and (3) applying an orientation treatment. In the form of the optical element according to the invention, it is preferred to dispose a layer containing the compound of the invention as at least a portion of the constituent component on a single substrate or between a pair of substrates.

In the step of dissolving a composition containing the compound of the invention and the polymeric medium supporting the same, or in the step of dissolving a composition containing the polymer of the compound of the invention into a solvent, the polymeric medium is not particularly limited. Examples thereof include acrylic polymer such as PMMA, imide type polymer such as fluoropolyimide and polyarbonate. Further, the solvent to be used is not particularly limited. Examples thereof include ester type solvent such as ethyl acetate, ketone type solvent such as methyl ethyl ketone, ether type solvent such as tetrahydrofuran and halogen type solvent such as chloroform and dichloromethane, as well as mixed solvents thereof.

As a method of disposing a layer containing a compound having a non-linear optical responsive group (a portion represented by D-$Ar^1$-thiophene ring-$Ar^2$-A in general formula (I) or $D^1$-$Ar^1$-thiophene ring-$Ar^2$-$P^4$ in general formula (I-A)) as at least a portion of the constituent component on a substrate, a well-known method is adopted.

Preferred examples of the substrate include transparent substrates such as glass, polymeric film or reflection plate, and a transparent electrode layer or insulation film may also be disposed optionally on the substrate. A transparent

electrode may or may not be provided but ITO vapor deposited on the substrate is preferred, with no particular restriction thereto. The insulation film may or may not be provided and an insulation film of polyimide type or polyvinyl alcohol is preferred when the insulation film is provided. Further, in a case of using a pair of substrates, spacer, sealant and the like may be used optionally.

A known method may be adopted for coating, and examples of the coating method include curtain coating, extrusion coating, roll coating, spin coating, dip coating, bar coating, spray coating, slide coating and printing coating.

In the orientation treatment, the molecules are oriented by applying external electric fields or external magnetic fields. As the orientation method, a method of using the external electric fields is preferred and use of contact poling method (plain electrode poling method, electrode sandwich poling method) or corona poling method is preferred.

When the compound of the invention has crosslinkable substituents, the polymer comprising the compound as one of the constituent components can be prepared by the following methods, which should not be construed to limit the scope of the invention. For forming the polymer, various known crosslinking methods suitable to introduced crosslinkable substituents can be adopted. For example, when the crosslinkable substituent is an acryloyloxy group or methacryloyloxy group, radical polymerization of using a radical initiator such as AIBN in an organic solvent is particularly preferred.

It is preferred that the compound of the invention has a liquid crystal property. When the compound of the invention shows the liquid crystal property, application of the external electric fields or external magnetic fields is conducted preferably within a temperature range showing the liquid crystal phase. Further, a method of heating to an isometric phase under the application of external electric fields or external magnetic fields and then cooling the same to a liquid crystal phase is also preferred. For the intensity of the external electric fields or external magnetic fields to be used, an appropriate intensity for the orientation control of liquid crystal molecules is used.

To the layer containing the crosslinkable compound as at least a portion of the constituent components, an appropriate polymerization initiator, polymerization inhibitor, photosensitizer, crosslinker, liquid crystal orientation aid, etc. may be added as required.

Figs. 1 and 2 to be described below show typical examples of a layer structure for the non-linear optical material in the invention. In the drawing, are shown a non-linear optical response layer 1, an insulation film 2, and a transparent electrode substrate 3.

Embodiments of the present invention will be described as follows.

A first embodiment of the invention provides a compound represented by the following general formula (I):

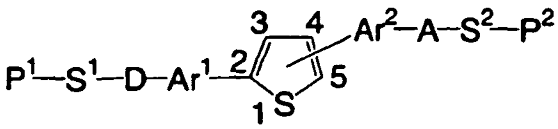

General Formula (I)

In general formula (I), $-Ar^2-A-S^2-P^2$ substitutes on the 4-position or 5-position of a thiophene ring. $Ar^1$ and $Ar^2$ each independently represent a single bond, an aromatic ring having 5 to 14 carbon atoms, or a biphenyl group, provided that at least one of $Ar^1$ and $Ar^2$ represents one of a naphthalene ring and a biphenyl group which may have a substituent. D represents an oxygen atom (-O-), a sulfur atom (-S-), a substituted amino group (-NR-) in which R represents a hydrogen atom, a lower alkyl group having 1 to 6 carbon atoms, or a substituent represented by $P^1-S^1-$, or an amino group having a cyclic structure. A represents an electron attracting group selected from an ester group (-COO-), a sulfonyl group ($-SO_2-$), and a sulfonyloxy group ($-SO_3-$). $S^1$ and $S^2$ each independently represent a bivalent linking group. $P^1$ and $P^2$ each independently represent a substituent consisting of atoms selected from C, H, O, N, S, and a halogen atom. At least one of $P^1$ and $P^2$ represents a polymerizable group. The thiophene ring may have a substituent.

A second embodiment of the invention provides the compound of the first embodiment, wherein $Ar^1$ represents a benzene ring and $Ar^2$ represents a naphthalene ring; or $Ar^1$ represents a naphthalene ring and $Ar^2$ represents a benzene ring.

A third embodiment of the invention provides the compound of the first or second embodiment, wherein $S^1$ and $S^2$ each independently represent an alkylene group having 2 to 12 carbon atoms.

A fourth embodiment of the invention provides the compound of any one of the first to third embodiments, wherein $P^1$ and $P^2$ each independently represent an acryloyloxy group or a methacryloyloxy group.

A fifth embodiment of the invention provides an optical element containing the compound of any one of the first to fifth embodiments or a polymer of the compound.

A sixth embodiment of the invention provides a non-linear optical material containing the compound of any one of the first to fifth embodiments, or a polymer formed by polymerizing the compound, as at least one constituent component.

An seventh embodiment of the invention provides an electro-optical material containing the compound of any one of the first to fifth embodiments, or a polymer formed by polymerizing the compound, as at least one constituent component.

A eighth embodiment of the invention provides a compound represents by the following general formula (I-A):

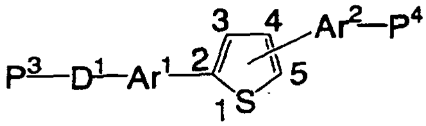

General Formula (I-A)

In general formula (I-A), $Ar^1$ and $Ar^2$ have the same meaning as descried in general formula (I). $-Ar^2-P^4$ substitutes on the 4-position or 5-position of a thiophene ring. $P^3$ represents a linear, branched or cyclic alkyl group having 1 to 20 carbon atoms wherein one or more of $-CH_2-$ in the alkyl group may be replaced with -O-, -CO-, $-NR^1-$ in which $R^1$ represents a linear, branched or cyclic alkyl group having 1 to 20 carbon atoms,-CH=CH-, or -C≡C-, provided that hetero atoms are not located adjacent to each other, and the alkyl group may have a substituent consisting of atoms selected from C, H, O, N, S, and a halogen atom. $P^4$ represents $-P^{4'}-P^6$ wherein $P^6$ represents a linear, branched or cyclic alkyl group having 1 to 20 carbon atoms wherein one or more of $-CH_2-$ in the alkyl group may be replaced with -O-, -CO-, -CH=CH-, or -C≡C-, provided that hetero atoms are not located adjacent to each other, and the alkyl group may have a substituent consisting of atoms selected from C, H, O, N, S, and a halogen atom, and $P^{4'}$ represents a bivalent electron attracting group selected from the group consisting of an ester group (-COO-), a sulfonyl group ($-SO_2-$), and a sulfoxy group ($-SO_3-$). $D^1$ represents one selected from the group consisting of an oxygen atom (-O-), a sulfur atom (-S-), and $-NP^5-$ in which $P^5$ represents a hydrogen atom or a linear, branched or cyclic alkyl group having 1 to 20 carbon atoms, wherein one or more of $-CH_2-$ in the alkyl group may be replaced with -O-, -CO-, -CH=CH-, or -C=C-, provided that hetero atoms are not located adjacent to each other, and the alkyl group may have a substituent consisting of atoms selected from C, H, O, N, S, and a halogen atom, and $P^5$ may be independent of $P^3$, or bonded with $P^3$ to form a ring. The thiophene ring may have a substituent.

A tenth embodiment of the invention provides the compound of the ninth embodiment, wherein $Ar^1$ is a 1,4-phenylene group and $Ar^2$ is 2,6-naphthalene group; or $Ar^1$ is a 2,6-naphthalene group and $Ar^2$ is a 1,4-phenylene group.

A eleventh embodiment of the invention provides the compound of the eleventh or twelfth embodiment, wherein $P^3$ and $P^5$ each independently represent a linear, branched, or cyclic alkyl group having 1 to 20 carbon atoms.

A twelfth embodiment of the invention provides an optical element containing the compound of the ninth to thirteenth embodiments or a polymer of the compound.

A thirteenth embodiment of the invention provides a non-linear optical material containing the compound of any one of ninth to thirteenth embodiments, or a polymer formed by polymerizing the compound, as at least one constituent component.

A fourteenth embodiments of the invention provides an electro-optical material containing the compound of any one of ninth to thirteenth embodiments, or a polymer formed by polymerizing the compound, as at least one constituent component.

EXAMPLES

The present invention is to be described more specifically by way of examples but the scope of the invention should not be limited thereto.

Synthesis Example 1:

Synthesis of 6-{5-[4-(2-acryloyloxy-ethoxy)-phenyl]-thiophen-2-yl}-naphthalene-2-carboxylic acid 2-acryloyloxy-ethyl ester (compound 1)

Compound 1

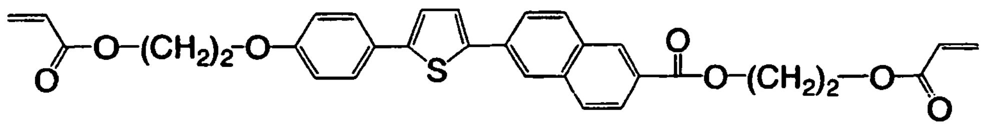

1-bromo-4-methoy-benzene (24.1 g, 128.9 mmol) and thiophene boronic acid (15.0g, 117.2 mmol) were dissolved in 280 ml of dimethylformamide (DMF) and stirred under the presence of $Pd(PPh_3)_4$ (1.35 g, 1.17 mmol) and potassium carbonate (40.5 g, 293 mmol), at 80°C for 10 hours. Then, 300 ml of water was added and extracted with ethyl acetate. After washing with an aqueous saturated solution of sodium chloride, the extracted solution was evaporated on anhydrous magnesium sulfate and then the solvent was distilled off by a rotary evaporator. Then, the residue was isolated by flush column chromatography using hexane/ethyl acetate = 20/1 mixed solvent, to obtain 2-(4-methoxyphenyl)-thiophene as white crystals (amount 19.8 g, yield 89.0%).

Then, 2-(4-methoxy-phenyl)-thiophene (13.5 g, 70.0 mmol) was dissolved in 120 ml of tetrahydrofuran (THF), to which n-butyl lithium (84.8 mmol, 1.6 M hexane solution) was added gradually at -78°C. After stirring as it was for 1 hour, triethyl borate ($B(OEt)_3$) was added and stirred at a room temperature for 2 hours. Then, an aqueous solution of diluted hydrochloric acid was added and extracted with ethyl acetate. After washing with an aqueous saturated solution of sodium chloride, the extracted solution was dried on anhydrous magnetic sulfate and the solvent was distilled off by a rotary evaporator, to obtain 5-(4-methoxyphenyl)-thiophene-2-boronic acid (14.9 g).

Then, 5-(4-methoxy-phenyl)-thiophene-2-boronic acid (5.42 g, 23.2 mmol) and 6-bromo-naphthalene-2-carboxylic acid methyl ester (6.15 g, 23.2 mmol) were dissolved in 65 ml of dimethylformamide (DMF) and stirred under the presence of $Pd(PPh_3)_4$ (266 mg, 0.23 mmol) and potassium carbonate (9.60 g, 69.6 mmol) at 80°C for 12 hours. Then, 150 ml of water was added and extracted with ethyl acetate. After cleaning with an aqueous saturated solution of sodium chloride, the extracted solution was dried on anhydrous magnesium sulfate and the solvent was distilled off by a rotary evaporator. Then, the residue was isolated by flush column chromatography using hexane/ethyl acetate = 1/ 1 mixed solvent, to obtain 6-[5-(4-methoxy-phenyl)-thiophen-2-yl]-naphthalene-2-carboxylic acid methyl ester (amount 5.2 g, yield 61%).

Then, 6-[5-(4-methoxy-phenyl)-thiophen-2-yl]-naphthalene-2-carboxylic acid methyl ester (4.10 g, 10.9 mmol) was dissolved in 85 ml of dichloromethane, to which 21.8 ml of 1.0 mol/L dichloromethane solution of boron tribromide ($BBr_3$) was dropped under ice cooling and then stirred at a room temperature for 4 hours. Then, water was added to the reaction solution, and resultant precipitates were filtered and washed with water to obtain 3.80 g of 6-[5-(4-hydroxy-phenyl)-thiophen-2-yl]-naphthalene-2-carboxylic acid.

The method of synthesizing the compound represented by P-S-L in the scheme 2 is shown. Ethylene glycol (18.6 g, 300 mmol) and diisopropyl ethylamine (58.2 g, 450 mmol) were dissolved in 500 ml of THF, and acrylic acid chloride (27.2 g, 300 mmol) was gradually dropped to the mixed solution. After stirring at a room temperature for 3 hours, 500 ml of water was added and extracted with ethyl acetate and washed with aqueous saturated solution of sodium chloride. Then, the extracted solution was dried on anhydrous magnesium sulfate and the solvent was distilled off. The residue was isolated by flush column chromatography using hexane/ethyl acetate = 3/1 mixed solvent, to obtain acrylic acid 2-hydroxy-ethyl ester (amount 23 g, yield 66%).

Acrylic acid 2-hydroxy-ethyl ester (20 g, 172 mmol) and triethylamine (34.8 g, 344 mmol) were dissolved in 250 ml of THF, and methane sulfonyl chloride (23.6 g, 206 mmol) was gradually dropped to the mixed solution. After stirring at a room temperature for 5 hours, 250 ml of water was added, extracted with ethyl acetate and washed with an aqueous saturated solution of sodium chloride. Then, extracted solution was dried on anhydrous magnesium sulfate and the solvent was distilled off to obtain a compound: acrylic acid 2-methanesulfonyloxy-ethyl ester represented by P-S-L in the scheme 2.

Finally, 6-[5-(4-hydroxy-phenyl)-thiophen-2-yl]-naphthalene-2-carboxylic acid (200 mg, 0.577 mmol) and acrylic acid 2-methanesulfonyloxy-ethyl ester (449 mg, 2.31 mmol) were heated in 5 ml of dimethylformamide under the presence of potassium carbonate (478 mg, 3.46 mmol) at 80°C and stirred for 8 hours. Then, water was added to the reaction solution, extracted with ethyl acetate and the extracted solution was washed with an aqueous saturated solution of sodium hydrogen carbonate and an aqueous saturated solution of sodium chloride. The extracted solution (organic layer) was dried on anhydrous magnesium sulfate. It was concentrated by a rotary evaporator and the concentrated solution was purified by silica gel chromatography (developing solution: hexane/ethyl acetate = 3/1) to synthesize a compound 1.

FAB-MS : $(M + H)^+ = 543$

When the obtained compound was observed under polarization microscope, it exhibited liquid crystal property and showed the following phase transition temperature.

In the following descriptions, C represents the crystal, N represents the nematic phase, S represents the smectic phase and I represents the isometric phase.

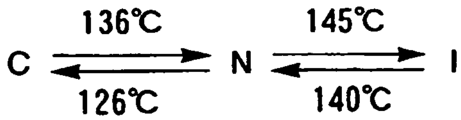


Synthesis Example 2:

Synthesis of 6-{5-[4-(4-acryloyloxy-butoxy)-phenyl]-thiophen-2-yl}-naphthalene-2-carboxylic acid 4-acryloyloxy-butyl ester (compound 2)

Compound 2

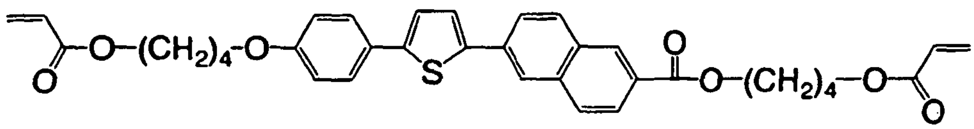

Acrylic acid 2-methanesulfonyloxy-ethyl ester was replaced with acrylic acid 4-methanesulfonyloxy-butyl ester and a compound 2 was synthesized in accordance with the method of Synthesis Example 1.

FAB-MS : $(M + H)^+ = 599$

When the obtained compound was observed under a polarization microscope, it exhibited a liquid crystal property and showed the following phase transition temperature.

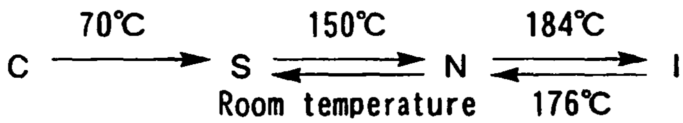


Synthesis Example 3:

Synthesis of 6-{5-{4-(6-acryloyloxy-hexyloxy)-phenyl]-thiophen-2-yl}-naphthalene-2-carboxylic acid 6-acryloyloxy-hexyl ester (compound 3)

Compound 3

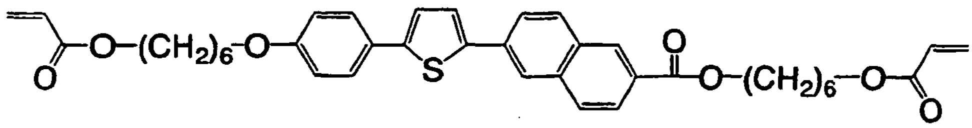

Acrylic acid 2-methanesulfonyloxy-ethyl ester was replaced with acrylic acid 6-methanesulfonyloxy-hexyl ester and the compound 3 was synthesized in accordance with the method of Synthesis Example 1.
FAB-MS : $(M + H)^+ = 655$

When the obtained compound was observed under a polarization microscope, it exhibited a liquid crystal property and showed the following phase transition temperature.

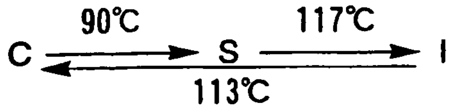


Synthesis Example 4:

Synthesis of 6-{5-[4-(12-acryloyloxy-dodecyloxy)-phenyl]-thiophen-2-yl}-naphthalene-2-carboxylic acid 12-acryloyloxy-dodecyl ester (compound 4)

Compound 4

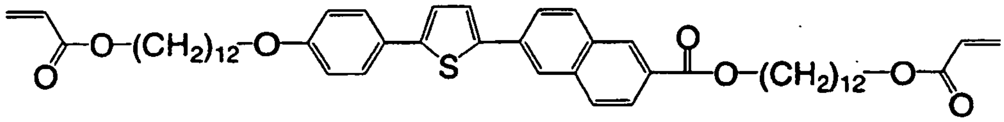

Acrylic acid 2-methanesulfonyloxy-ethyl ester was replaced with acrylic acid 12-methanesulfonyloxy-dodecyl ester and the compound 4 was synthesized in accordance with the method of Synthesis Example 1.
FAB-MS : $(M + H)^+ = 823$

Synthesis Example 5:

Synthesis of 6-{5-[4-(4-acryloyloxy-butoxy)-phenyl]-thiophen-2-yl}-naphthalene-2-carboxylic acid 6-acryloyloxy-hexyl ester (compound 5)

Compound 5

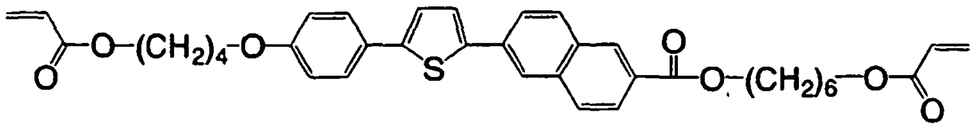

6-[5-)4-hydroxy-phenyl)-thiophen-2-yl]-naphthalene-2-carboxylic acid (2.0 g, 5.77 mmol) was dissolved in 100 ml of tetrahydrofuran (THF), to which thionyl chloride (820 mg, 6.92 mmol) was added. After stirring at a room temperature for 3 hours, acrylic acid 6-hydroxy-hexyl ester (1.19 g, 6.92 mmol) and triethylamine (1.40 g, 13.8 mmol) were added and stirred at 70°C for 4 hours. A phosphoric acid buffer was added and extracted with ethyl acetate. After cleaning the extracted solution with an aqueous saturated solution of sodium chloride it was dried on anhydrous magnesium sulfate. After distilling off the solvent, it was purified on silica gel chromatography (developing solution: hexane/ethyl acetate = 2/ 1), to obtain 6-[5-(4-hydroxy-phenyl-thiophen-2-yl]-naphthalene-2-carboxylic acid 6-acryloyloxy hexyl ester (1.73 g, 66% yield).

Then, 6-[5-(4-hydroxy-phenyl)-thiophen-2-yl]-naphthalene-2-carboxylic acid 6-acryloyloxy-hexyl ester (1.73 g, 3.46 mmol) and acrylic acid 4-methanesulfonyloxy-butyl ester (1.53 g, 6.92 mmol) were heated in dimethylformamide under the presence of potassium carbonate (1.43 g, 10.4 mmol) at 80°C and stirred for 5 hours. Then, water was added to the reaction solution, extracted with ethyl acetate and the extracted solution was washed with an aqueous saturated solution of sodium hydrogen carbonate and an aqueous saturated solution of sodium chloride. The extracted solution (organic layer) was dried on anhydrous magnesium sulfate. It was concentrated by a rotary evaporator and the concentrated solution was purified on silica gel chromatography (developing solution: hexane/ethyl acetate = 3/ 1) to synthesize the compound 5.

FAB-MS : $(M + H)^+ = 627$

Synthesis Example 6:

Synthesis of 6-{5-[4-(8-acryloyloxy-octyloxy)-2-methyl-phenyl]-thiophen-2-yl]-naphthalene-2-carboxylic acid 8-acryloyloxy-octyl ester (compound 8)

Compound 8

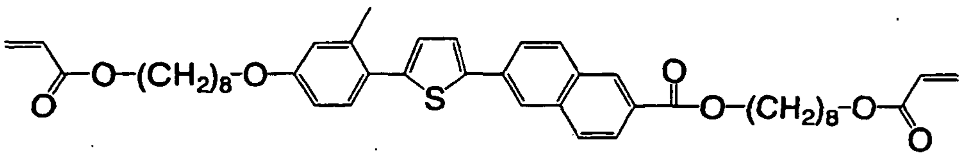

1-bromo-4-methoxy-benzene was replaced with 4-bromo-1-methoxy-2-methyl-benzene, and acrylic acid 2-methanesulfonyloxy-ethyl ester was replaced with acrylic acid 8-methanesulfonyloxy-octyl ester and the compound 8 was synthesized in accordance with the method of Synthesis Example 1.

FAB - MS: $(M + H)^+ = 725$

$^1$H NMR ($CDCl_3$, 300MHz, δ) : 1.35-1.49 (m, 24 H), 1.66-1.71 (m, 4 H), 1.80-1.83 (m, 4H), 3.97-4.00 (t, J = 6.6 Hz, 2 H), 4.14-4.18 (t, J = 6.6 Hz, 4 H), 4.37-4.39 (t, J = 6.6 Hz, 2 H), 5.80-5.84 (dd, J = 9.6, 1.8 Hz, 2 H), 6.10-6.20 (dd, J = 13.2, 1.8 Hz, 2 H), 6.37-6.42 (dd, J = 10.2, 1.8 Hz, 2 H), 6.92-6.95 (d, J = 9.0 Hz, 2 H), 7.22-7.24 (d, J = 3.9 Hz, 1 H), 7.44-7.45 (d, J = 2.7 Hz, 1 H), 7.56-7.59 (d, J = 8.7 Hz, 2 H), 7.80-7.88 (dd, J = 8.6, 4.3 Hz, 1 H), 7.94-7.97 (d, J = 4.3 Hz, 1 H), 8.06 (s, 2 H), 8.56 (s, 1 H)

When the obtained compound was observed under a polarization microscope, it exhibited the liquid crystal property and showed the following phase transition temperature.

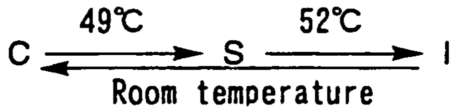


Synthesis Example 7:

Synthesis of 4-{5-[6-(6-acryloyloxy-hexyloxy)-naphthalen-2-yl]-thiophen-2-yl}-benzoic acid 6-acryloyloxy-hexyl ester (compound 13)

Compound 13

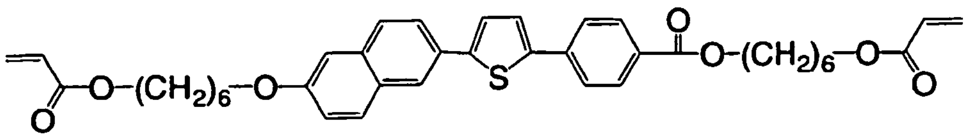

1-bromo-4-methoxy-benzene was replaced with 2-bromo-6-methoxy-naphthalene, 6-bromo-naphthalene-2-carboxylic acid methyl ester was replaced with 4-bromo-benzoic acid methyl ester and acrylic acid 2-methanesulfonyloxy-ethyl ester was replaced with acrylic acid 6-methanesulfonyloxy-hexyl ester, and the compound 13 was synthesized in accordance with the method of Synthesis Example 1.
FAB-MS: $(M + H)^+ = 655$

Synthesis Example 8:

Synthesis of 4'-{5-[4-(6-acryloyloxy-hexyloyloxy)-phenyl]-thiophen-2-yl}-biphenyl-4-carboxylic acid 6-acryloyloxy-hexyl ester (compound 15)

Compound 15

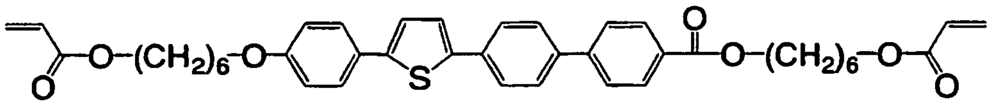

6-bromo-naphthalene-2-carboxylic acid methyl ester was replaced with 4'-bromo-biphenyl-4-carboxylic acid methyl ester and a compound 15 was synthesized in accordance with the method of Synthesis Example 3.
FAB - MS: $(M + H)^+ = 681$

Synthesis Example 9:

Synthesis of 6-(5-{4-[(6-acryloyloxy-hexyl)methyl-amino]-phenyl} thiophen-2-yl)-naphthalene-2-carboxylic acid 6-acryloyloxy-hexyl ester (compound 17)

Compound 17

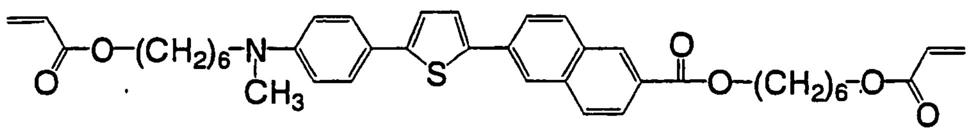

At first, 1-bromo-4-methoxy-benzene was replaced with N-(4-bromo-phenyl)-N-methyl-acetamide and 6-{5-{4-(acetyl-methyl-amino)-phenyl]-thiophen-2-yl}-naphthalene-2-carboxylic acid methyl ester was synthesized in accordance with the method of Synthesis Example 1.

6-{5-{4-(acetyl-methyl-amino)-phenyl]-thiophen-2-yl}-naphthalene-2-carboxylic acid methyl ester (4.3 g, 10.3 mmol) were dissolved in 100 ml of dimethyl sulfoamide, to which 10 ml of 5.0 N hydrochloric acid and 20 ml of water were added and stirred at 80°C for 12 hours. Then, water was added to the reaction solution and precipitates were separated by filtration. After washing the precipitates with ethyl acetate, an aqueous saturated solution of sodium hydrogen carbonate and purified water, they were dried by heating under vacuum to obtain 3.38 g of 6-[5-(4-methylaminophenyl)-thiophen-2-yl]-naphthalene-2-carboxylic acid.

Finally, 6-[5-(4-methylamino-phenyl)-thiophen-2-yl]-naphthalene-2-carboxylic acid (1.00 g, 2.78 mmol) and acrylic acid 2-methanesulfonyloxy-hexyl ester (2.78 g, 11.1 mmol) were heated at 80°C in 1 ml of dimethylformamide under the presence of potassium carbonate (2.30 g, 16.7 mmol) and stirred for 10 hours. Then water was added to the reaction solution, and extracted with ethyl acetate and the extracted solution was washed with an aqueous saturated solution of sodium hydrogen carbonate and an aqueous saturated solution of sodium chloride. The extracted solution (organic layer) was dried on anhydrous magnetic sulfate. It was concentrated by a rotary evaporator and the concentrated solution was purified on silica gel chromatography (developing solution: hexane / ethyl acetate = 2/1) to synthesize the compound 17.

FAB-MS: $(M + H)^+ = 668$

Synthesis Example 10:

Synthesis of 6-(5-{4-(bis-(6-acryloyloxy-hexyl)-amino]-phenyl}-thiophen-2-yl)-naphthalene-2-carboxylic acid 6-acryloyloxy-hexyl ester (compound 19)

Compound 19

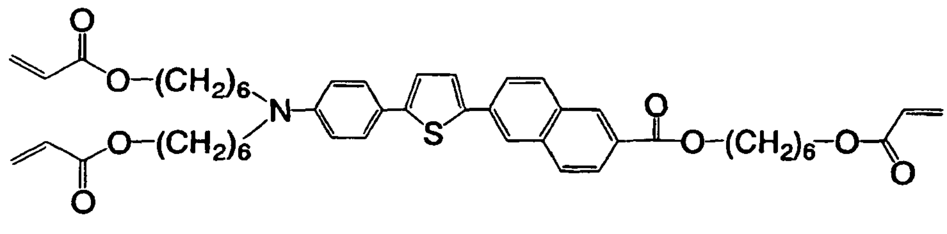

At first, N-(4-bromo-phenyl)-N-methyl-acetamide was replaced with N-(4-bromo-phenyl-acetamide, and 6-[5-(4-acetylamino-phenyl)-thiophen-2-yl]-naphthalene-2-carboxylic acid methyl ester was synthesized in accordance with the method of Synthesis Example 9.

Then, 6-[5-(4-acetylamino-phenyl)-thiophen-2-yl]-naphthalene-2-carboxylic acid methyl ester (1.5 g, 3.74 mmol) was dissolved in 30 ml of dimethylformamide, to which 5 ml of 5.0 N hydrochloric acid and 15 ml of water were added and stirred at 100°C for 24 hours. Then, water was added to the reaction solution and resultant precipitates were separated by filtration. After washing the precipitates with ethyl acetate, an aqueous saturated solution of sodium hydrogen carbonate and purified water, they were dried by heating under vacuum to obtain 6-[5-(4-amino-phenyl)-thiophen-2-yl]-naphthalene-2-carboxylic acid.

Finally, 6-[5-(4-amino-phenyl)-thiophen-2-yl]-naphthalene-2-carboxylic acid (0.6 g, 1.73 mmol) and acrylic acid

6-methanesulfonyloxy-hexyl ester (2.6 g, 10.4 mmol) were heated at 80°C in 10 ml of dimethylformamide under the presence of potassium carbonate (1.43 g, 10.4 mmol) and stirred for 12 hours. Then water was added to the reaction solution and extracted with ethyl acetate, and the extracted solution was washed with an aqueous saturated solution of sodium hydrogen carbonate and an aqueous saturated solution of sodium chloride. The extracted solution (organic layer) was dried on anhydrous magnetic sulfate. It was concentrated by a rotary evaporator and the concentrated solution was purified on silica gel chromatography (developing solution: hexane / ethyl acetate = 3/1) to synthesize the compound 19.
FAB-MS: $(M + H)^+ = 808$

Synthesis Example 11:

Synthesis of 6-(5-{4-[4-(acryloyloxy-hexyloxy)-piperidin-1-yl]-phenyl}-thiophen-2-yl)-naphthalene-2-carboxylic acid 6-acryloyloxy-hexyl ester (compound 20)

Compound 20

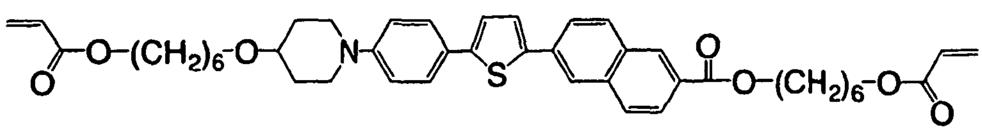

4-methoxy-piperidine (12.7 g, 110 mmol) and 1-bromo-4-fluorobenzene (19.3 g, 110 mmol) were dissolved in 150 ml of dimethylsulfoxide (DMSO) and stirred at 100°C for 10 hours under the presence of potassium carbonate (22.8 g, 165 mmol). After adding 200 ml of water, it was extracted with ethyl acetate and washed with an aqueous saturated solution of sodium chloride. The extracted solution (organic layer) was dried on anhydrous magnesium sulfate and then the solvent was distilled off. Then, the concentrated solution was purified on silica gel chromatography (developing solution: hexane/ethyl acetate = 1/2), to obtain 1-(4-bromo-phenyl)-4-methoxy-piperidine (amount 17.5 g, yield 59%).
Then, 1-bromo-4-methoxy-benzene was replaced with 1-(4-bromophenyl)-4-methoxy-piperidine and the compound 20 was synthesized in accordance with the method of Synthesis Example 1.
FAB-MS : $(M + H)^+ = 738$

Synthesis Example 12

Synthesis of compound 23

6-[-5-(4-hydroxy-2-methyl-phenyl)-thiophen-2-yl]-naphthalene-3-carboxylic acid was synthesized by the method described in Synthetic Example 6.
Then, 6-[-5-(4-hydroxy-2-methyl-phenyl)-thiophen-2-yl]-naphthalene-2-carboxylic acid obtained (1.4 g, 3.9 mmol) and methanesulfonic acid decyl ester (2.76 g, 11.7 mmol) were heated to 70°C in 20 ml of dimethyl formamide under the presence of potassium carbonate (2.1 g, 15 mmol) and stirred for 10 hours. Then, water was added to the reaction solution and extracted with ethyl acetate, and the extracted solution was washed with an aqueous saturated solution of sodium hydrogen carbonate and an aqueous saturated solution of sodium chloride. The extracted solution (organic layer) was dried on anhydrous magnesium sulfate. It was concentrated by a rotary evaporator and the concentrated solution was purified on silica gel chromatography (developing solution: hexane/ethyl acetate = 5/ 1), to obtain the compound 23 (1.8 g, yield 62%).
FAB-MS: $(M + H)^+ = 641$
When the resultant compound 23 was observed under polarization microscope, it exhibited the liquid crystalline property and showed the following phase transition temperature. Further, $S_C$ represents the smectic phase C and $S_A$ represents the smectic phase A.

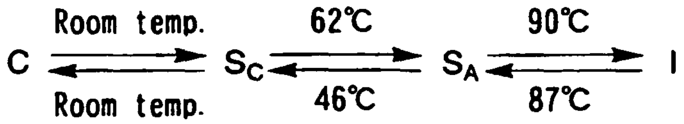


Synthesis Example 13

Synthesis of compound 24

At first, 4-bromo-2-methyl-phenylamine (25 g, 134 mmol) and 1-bromo-octane (26 g, 134 mmol) were stirred in DMSO under the presence of potassium carbonate (37 g, 268 mmol). Then, 150 ml of water was added and extracted with ethyl acetate. After washing with an aqueous saturated solution of sodium chloride, the extracted solution was dried on anhydrous magnesium sulfate and the solvent was distilled off by a rotary evaporator. Then, the residue was purified on flush column chromatography using hexane/ethyl acetate = 5/ 1 mixed solvent, to obtain (4-bromo-2-methyl-phenyl)-octyl-amine (28.7 g, yield 72%).

Then, (4-bromo-2-methyl-phenyl)-methyl-octyl-amine (28.7 g, 96.2 mmol) was stirred with iodomethane (27 g, 192 mmol), to obtain (4-bromo-2-methyl-phenyl)-methyl-octyl-amine (25 g, yield 85%). Purification was conducted on flush column chromatography (developing solution : hexane/ethyl acetate = 10/1).

Then, 1-bromo-4-methoxy-benzene was replaced with (4-bromo-2-methyl-phenyl)-methyl-octyl-amine (25 g, 81 mmol), and 5-[3-methyl-4-(methyl-octyl-amino)-phenyl]-thiophene-2-boronic acid was synthesized in accordance with the method of Synthesis Example 1. The obtained 5-[3-methyl-4-methyl-octyl-amino)-phenyl]-thiophene-2-boronic acid (5.4 g, 15 mmol) and 6-bromo-naphthalene-2-carboxylic acid octyl ester (5.8 g, 16 mmol) were dissolved in 30 ml of DMF and stirred under the presence of $Pd(PPh_3)_4$ (184 mg, 0.16 mmol) and potassium carbonate (4.4 g, 32 mmol) and stirred for 12 hours at 80°C. Then, 50 ml of water was added and extracted with ethyl acetate. After cleaning with an aqueous saturated solution of sodium chloride, the extracted solution was dried on anhydrous magnesium sulfate and the solvent was distilled off by a rotary evaporator. Then, it was isolated on flush column chromatography using hexane/ ethyl acetate =1/1 mixed solvent, to obtain the aimed compound 24.
FAB-MS $(M + H)^+ = 598$

Other compounds of the invention can also be synthesized in accordance with the method of Synthesis Example 1.

Example 1

A constituent material comprising 6-{5-[4-(4-acryloyloxy-butoxy)phenyl]-thiophen-2-yl}-naphthalene-2-carboxylic acid 4-acryloyloxy-butyl ester (compound 2) (92.7 parts by weight), phenothiazine (1.3 parts by weight), a polymerization initiator (Illugacure 651, trade name of products manufactured by Ciba Guigy) (4 parts by weight) and hydroquinone monomethyl ether (2 parts by weight) was sandwiched in a horizontally oriented cell having a 4 μm gap and comprising a glass substrate having ITO transparent electrode portions formed by coating a polyimide thin film as an insulation film (manufactured by E. H. I). Then, the obtained sample was kept at 160°C while applying a DC voltage at 200 V across transparent electrodes, and UV irradiation (254 nm, 10 W/cm, 3 min) was conducted.

Infrared light of YAG laser (1.6 μm) was irradiated to the sample obtained as described above to confirm generation of second harmonic waves. The intensity of the second harmonic waves of the sample was maintained for one month.

Example 2

A chloroform solution comprising 6-{5-[4-(4-acryloyloxy-butoxy)-phenyl]-thiophen-2-yl}-naphthalene-2-carboxylic acid 4-acryloyloxy-butyl ester (compound 2) (92.7 parts by weight), phenothiazine (1.3 parts by weight), a polymerization initiator (Illugacure 651, trade name of products manufactured by Ciba Guigy Co.) (4 parts by weight), and hydroquinone monomethyl ether (2 parts by weight) was coated by spin coating (1000 rpm, 20 sec) on a glass substrate having ITO transparent electrode portions formed by coating a polyimide film as an insulation film and then dried under a reduced pressure for 12 hours. Then, the obtained sample was kept at 160°C and voltage was applied by using a corona poling method (application voltage: 5.0 kV, 20 min) and then UV irradiation (254 nm, 10 W/cm, 3 min) was conducted while applying the voltage. An aluminum electrode was carried on a crosslinked non-linear optical response portion by vapor deposition to manufacture a non-linear optical material having electrodes on both ends.

Infrared light of YAG laser (1.06 μm) was irradiated to the sample obtained as described above to confirm the generation of second harmonic waves and the electro-optical effect. The intensity of the second harmonic waves and

the electro-optical effect of the sample were maintained for one month.

Fig. 3 shows the intensity of the second harmonic waves before and after corona poling for the sample obtained as described above.

From the examples describes above, it can be seen that the crosslinkable compound of the invention is useful as an organic non-linear optical material with remarkably suppressed aging decay for the electro-optical effect or generation of second harmonic waves.

Example 3

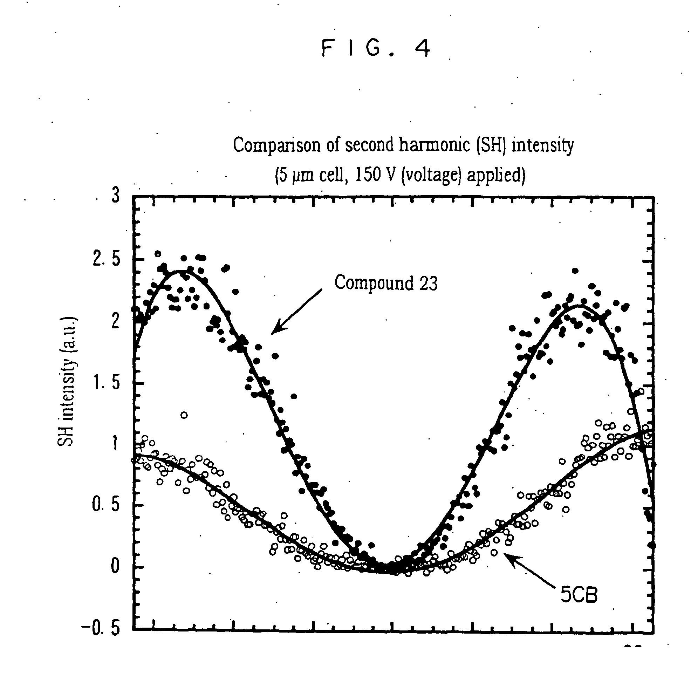

The compound 23 was sandwiched in a horizontally oriented cell having 5 μm gap and comprising a glass substrate with an ITO transparent electrode portion (manufactured by E. H. I). Then, the obtained sample was kept at 70°C while applying a DC voltage at 150 V across the transparent electrodes. An infrared light of YAG laser (1.06 μm) was irradiated to the obtained sample to confirm the generation of second harmonic waves. Fig. 4 shows the intensity of the second harmonic waves for the sample obtained as described above and for the sample of 5CB for reference use oriented under electric fields under the same conditions.

In view of the example, it can be seen that the non-linear optical compound of the invention has higher non-linear optical characteristics than the rod-like liquid crystal compound 5CB (compound for reference) in a state of electric field orientation and it is useful as an organic non-linear optical material.

The present invention can provide (1) a novel compound which is useful as a non-linear optical material comprising an organic material with no or controlled orientation relaxation, (2) a novel compound which is useful as a non-linear optical material that generates second harmonic waves upon electric field orientation and (3) an optical element, a non-linear optical material and an electro-optical material using the crosslinked product or non-crosslinked product of the compound.

## Claims

**1.** A compound represented by the following general formula (I):

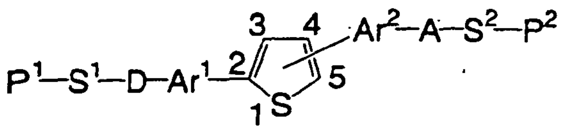

General Formula (I)

wherein:

$-Ar^2-A-S^2-P^2$ substitutes on the 4-position or 5-position of a thiophene ring;
$Ar^1$ and $Ar^2$ each independently represent a single bond, an aromatic ring having 5 to 14 carbon atoms, or a biphenyl group, provided that at least one of $Ar^1$ and $Ar^2$ represents one of a naphthalene ring and a biphenyl group which may have a substituent;
D represents an oxygen atom (-O-), a sulfur atom (-S-), a substituted amino group (-NR-) in which R represents a hydrogen atom, a lower alkyl group having 1 to 6 carbon atoms, or a substituent represented by $P^1-S^1-$, or an amino group having a cyclic structure; and
A represents an electron attracting group selected from an ester group (-COO-), a sulfonyl group ($-SO_2-$), and a sulfonyloxy group ($-SO_3-$);
$S^1$ and $S^2$ each independently represent a bivalent linking group;
$P^1$ and $P^2$ each independently represent a substituent consisting of atoms selected from C, H, O, N, S, and a halogen atom;
at least one of $P^1$ and $P^2$ represents a polymerizable group including an acryloyloxy group or a methacryloyloxy group; and
the thiophene ring may have a substituent.

**2.** The compound of claim 1, wherein:

$Ar^1$ represents a benzene ring and $Ar^2$ represents a naphthalene ring; or
$Ar^1$ represents a naphthalene ring and $Ar^2$ represents a benzene ring.

**3.** The compound of claim 1 or 2, wherein $S^1$ and $S^2$ each independently represent an alkylene group having 2 to 12 carbon atoms.

**4.** An optical element comprising the compound of any one of claims 1 to 3 or a polymer of the compound.

**5.** A non-linear optical material comprising the compound of any one of claims 1 to 3 or a polymer formed by polymerizing the compound, as at least one constituent component.

**6.** An electro-optical material comprising the compound of claims 1 to 3 or a polymer formed by polymerizing the compound, as at least one constituent component.

**7.** A compound represented by the following general formula (I-A):

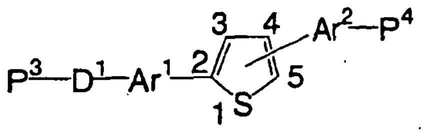

General Formula (I-A)

wherein:

> $Ar^1$ and $Ar^2$ each independently represent a single bond, an aromatic ring having 5 to 14 carbon atoms, or a biphenyl group, provided that at least one of $Ar^1$ and $Ar^2$ represents one of a naphthalene ring and a biphenyl group which may have a substituent;
> $-Ar^2-P^4$ substitutes on the 4-position or 5-position of a thiophene ring;
> $P^3$ represents a linear, branched, or cyclic alkyl group having 1 to 20 carbon atoms wherein one or more of $-CH_2-$ in the alkyl group may be replaced with -O-, -CO-, $-NR^1-$ in which $R^1$ represents a linear, branched or cyclic alkyl group having 1 to 20 carbon atoms, -CH=CH-, or -C≡C-, provided that hetero atoms are not located adjacent to each other, and the alkyl group may have a substituent consisting of atoms selected from C, H, O, N, S, and a halogen atom;
> $P^4$ represents $-P^{4'}-P^6$

wherein:

> $P^6$ represents a linear, branched, or cyclic alkyl group having 1 to 20 carbon atoms wherein one or more of $-CH_2-$ in the alkyl group may be replaced with -O-, -CO-, -CH=CH-, or -C=C-, provided that hetero atoms are not located adjacent to each other, and the alkyl group may have a substituent consisting of atoms selected from C, H, O, N, S, and a halogen atom; and
> $P^{4'}$ represents a bivalent electron attracting group selected from the group consisting of an ester group (-COO-), a sulfonyl group ($-SO_2-$), and a sulfoxy group ($-SO_3-$);
> $D^1$ represents one selected from the group consisting of an oxygen atom (-O-), a sulfur atom (-S-), and $-NP^5-$ in which $P^5$ represents a hydrogen atom or a linear, branched, or cyclic alkyl group having 1 to 20 carbon atoms, wherein one or more of $-CH_2-$ in the alkyl group may be replaced with -O-, -CO-, -CH=CH-, or -C=C-, provided that hetero atoms are not located adjacent to each other, and the alkyl group may have a substituent consisting of atoms selected from C, H, O, N, S, and a halogen atom; and $P^5$ may be independent of $P^3$, or bonded with $P^3$ to form a ring; and
> the thiophene ring may have a substituent.

**8.** The compound of claim 7, wherein:

> $Ar^1$ is a 1,4-phenylene group and $Ar^2$ is 2,6-naphthalene group; or
> $Ar^1$ is a 2,6-naphthalene group and $Ar^2$ is a 1,4-phenylene group.

**9.** The compound of claim 7 or 8, wherein $P^3$ and $P^5$ each independently represent a linear, branched, or cyclic alkyl group having 1 to 20 carbon atoms.

**10.** An optical element comprising the compound of any one of claims 7 to 9 or a polymer of the compound.

**11.** A non-linear optical material comprising the compound of any one of claims 7 to 9, or a polymer formed by polymerizing the compound, as at least one constituent component.

**12.** An electro-optical material comprising the compound of any one of claims 7 to 9, or a polymer formed by polymerizing the compound, as at least one constituent component.

**Patentansprüche**

**1.** Verbindung, die durch die folgende allgemeine Formel (I) dargestellt wird:

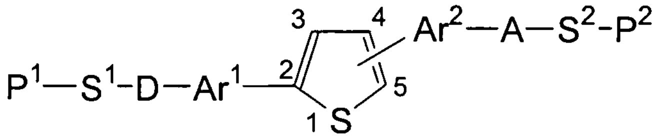

(I)

wobei:

-$Ar^2$-A-$S^2$-$P^2$ die 4-Position oder 5-Position eines Thiophenrings substituiert;
$Ar^1$ und $Ar^2$ jeweils unabhängig voneinander eine Einfachbindung, einen aromatischen Ring mit 5 bis 14 Kohlenstoffatomen oder eine Biphenylgruppe darstellen, unter der Voraussetzung, daß wenigstens eines von $Ar^1$ und $Ar^2$ eines aus einem Naphthalinring und einer Biphenylgruppe darstellen, die einen Substituenten aufweisen können;
D ein Sauerstoffatom (-O-), ein Schwefelatom (-S-), eine substituierte Aminogruppe (-NR-), in der R ein Wasserstoffatom, eine niedere Alkylgruppe mit 1 bis 6 Kohlenstoffatomen darstellt, oder einen durch $P^1$-$S^1$- dargestellten Substituenten oder eine Aminogruppe mit einer cyclischen Struktur darstellen; und
A eine elektronenanziehende Gruppe ausgewählt aus einer Estergruppe (-COO-), einer Sulfonylgruppe ($-SO_2-$) und einer Sulfonyloxygruppe ($-SO_3-$) darstellt;
$S^1$ und $S^2$ jeweils unabhängig voneinander eine divalente Verbindungsgruppe darstellen;
$P^1$ und $P^2$ jeweils unabhängig voneinander einen Substituenten bestehend aus Atomen ausgewählt aus C, H, 0, N, S und einem Halogenatom darstellen;
wenigstens eines von $P^1$ und $P^2$ eine polymerisierbare Gruppe darstellen, die eine Acryloyloxygruppe oder eine Methacryloyloxygruppe einschließen; und
der Thiophenring einen Substituenten aufweisen kann.

**2.** Verbindung gemäß Anspruch 1, wobei:

$Ar^1$ einen Benzolring darstellt und $Ar^2$ einen Naphthalinring darstellt; oder
$Ar^1$ einen Naphthalinring darstellt und $Ar^2$ einen Benzolring darstellt.

**3.** Verbindung gemäß Anspruch 1 oder 2, wobei $S^1$ und $S^2$ jeweils unabhängig voneinander eine Alkylengruppe mit 2 bis 12 Kohlenstoffatomen darstellen.

**4.** Optisches Element umfassend die Verbindung gemäß einem der Ansprüche 1 bis 3 oder ein Polymer dieser Verbindung.

**5.** Nichtlineares optisches Material umfassend als wenigstens einen Bestandteil die Verbindung gemäß einem der Ansprüche 1 bis 3 oder ein Polymer, das durch Polymerisieren der Verbindung gebildet wird.

**6.** Elektrooptisches Material umfassend als wenigstens einen Bestandteil die Verbindung gemäß einem der Ansprüche 1 bis 3 oder ein Polymer, das durch Polymerisieren der Verbindung gebildet wird.

**7.** Verbindung, die durch die folgende allgemeine Formel (I-A) dargestellt wird:

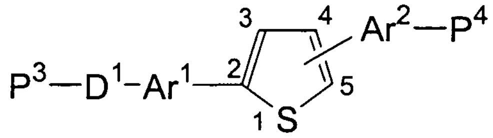

(I-A)

wobei:

Ar[1] und Ar[2] jeweils unabhängig voneinander eine Einfachbindung, einen aromatischen Ring mit 5 bis 14 Kohlenstoffatomen oder eine Biphenylgruppe darstellen, unter der Voraussetzung, daß wenigstens eines von Ar[1] und Ar[2] einen Naphthalinring und eine Biphenylgruppe darstellen, die einen Substituenten aufweisen können;
-$Ar^2$-$P^4$ die 4-Position oder 5-Position eines Thiophenrings substituiert;
$P^3$ eine lineare, verzweigte oder cyclische Alkylgruppe mit 1 bis 20 Kohlenstoffatomen darstellt, wobei eines oder mehrere der -$CH_2$- in der Alkylgruppe durch -O-, -CO-, -$NR^1$- ersetzt sein können, indem $R^1$ eine lineare, verzweigte oder cyclische Alkylgruppe mit 1 bis 20 Kohlenstoffatomen, -CH=CH- oder -C≡C- darstellt, vorausgesetzt, daß Heteroatome nicht benachbart zueinander angeordnet sind, und die Alkylgruppe kann einen Substituenten aufweisen, der aus den Atomen ausgewählt aus C, H, O, N, S und einem Halogenatom besteht;
$P^4$ -$P^{4'}$-$P^6$ darstellt,

wobei:

$P^6$ eine lineare, verzweigte oder cyclische Alkylgruppe mit 1 bis 20 Kohlenstoffatomen darstellt, wobei eines oder mehrere der -$CH_2$- in der Alkylgruppe durch -O-, -CO-, -CH=CH- oder -C=C- ersetzt sein können, vorausgesetzt, daß Heteroatome nicht benachbart zueinander angeordnet sind, und die Alkylgruppe kann einen Substituenten aufweisen, der aus den Atomen ausgewählt aus C, H, 0, N, S und einem Halogenatom besteht; und
$P^{4'}$ eine divalente elektronenanziehende Gruppe darstellt, die ausgewählt ist aus der Gruppe bestehend aus einer Estergruppe (-COO-), einer Sulfonylgruppe (-$SO_2$-) und einer Sulfoxygruppe (-$SO_3$-);
$D^1$ eines ausgewählt aus der Gruppe bestehend aus einem Sauerstoffatom (-O-), einem Schwefelatom (-S-) und -$NP^5$- darstellt, in dem $P^5$ ein Wasserstoffatom oder eine lineare, verzweigte oder cyclische Alkylgruppe mit 1 bis 20 Kohlenstoffatomen darstellt, wobei eines oder mehrere der -$CH_2$- in der Alkylgruppe durch -O-, -CO-, -CH=CH- oder -C≡C- ersetzt sein können, vorausgesetzt, daß Heteroatome nicht benachbart zueinander angeordnet sind, und die Alkylgruppe kann einen Substituenten aufweisen, der aus den Atomen ausgewählt aus C, H, O, N, S und einem Halogenatom besteht, und $P^5$ kann unabhängig von $P^3$ sein oder unter Ausbildung eines Rings an $P^3$ gebunden sein; und
der Thiophenring einen Substituenten aufweisen kann.

8. Verbindung gemäß Anspruch 7, wobei:

Ar[1] eine 1,4-Phenylengruppe ist und Ar[2] eine 2,6-Naphthalingruppe ist; oder
Ar[1] eine 2,6-Naphthalingruppe ist und Ar[2] eine 1,4-Phenylengruppe ist.

9. Verbindung gemäß Anspruch 7 oder 8, wobei $P^3$ und $P^5$ jeweils unabhängig voneinander eine lineare, verzweigte oder cyclische Alkylgruppe mit 1 bis 20 Kohlenstoffatomen darstellen.

10. Optisches Element umfassend die Verbindung gemäß einem der Ansprüche 7 bis 9 oder ein Polymer dieser Verbindung.

11. Nichtlineares optisches Material umfassend als wenigstens einen Bestandteil die Verbindung gemäß einem der Ansprüche 7 bis 9 oder ein Polymer, das durch Polymerisieren der Verbindung gebildet wird.

12. Elektrooptisches Material umfassend als wenigstens einen Bestandteil die Verbindung gemäß einem der Ansprüche 7 bis 9 oder ein Polymer, das durch Polymerisieren der Verbindung gebildet wird.

**Revendications**

1. Un composé représenté par la formule générale (I) :

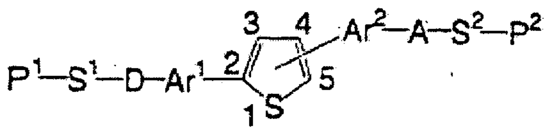

Formule générale (I)

dans laquelle :

- $Ar^2$ - A - $S^2$ - $P^2$ se substitue sur la position 4 ou la position 5 d'un anneau thiophène ;
$Ar^1$ et $Ar^2$ représentent chacun indépendamment une liaison simple, un anneau aromatique ayant de 5 à 14 atomes de carbone, ou un groupe biphényle, à condition qu'au moins l'un de $Ar^1$ et $Ar^2$ représente l'un d'un anneau naphthalène et d'un groupe biphényle qui peuvent avoir un substituant ;
D représente un atome d'oxygène (- O -), un atome de soufre (- S -), un groupe amino substitué (- NR -) dans lequel R représente un atome d'hydrogène, un groupe alcoyel inférieur ayant de 1 à 6 atomes de carbone, ou un substituant représenté par $P^1$ - $S^1$ -, ou un groupe amino ayant une structure cyclique ; et
A représente un groupe attracteur d'électrons sélectionné parmi un groupe ester (- COO -), un groupe sulfonyle (- $SO_2$ -), et un groupe sulfonyloxy (- $SO_3$ -) ;
$S^1$ et $S^2$ représentent chacun indépendamment un groupe de liaison bivalent ;
$P^1$ et $P^2$ représentent chacun indépendamment un substituant constitué d'atomes sélectionnés parmi C, H, O, N, S, et un atome d'halogène ;
au moins l'un de $P^1$ et $P^2$ représente un groupe polymérisable incluant un groupe acryloyloxy ou un groupe méthacryloyloxy ; et
l'anneau thiophène peut avoir un substituant.

**2.** Le composé de la revendication 1, dans lequel :

$Ar^1$ représente un anneau benzène et $Ar^2$ représente un anneau naphtalène ; ou
$Ar^1$ représente un anneau naphtalène et $Ar^2$ représente un anneau benzène.

**3.** Le composé de la revendication 1 ou 2, dans lequel $S^1$ et $S^2$ représentent chacun indépendamment un groupe alcoylène ayant de 2 à 12 atomes de carbone.

**4.** Un élément optique comprenant le composé de l'une quelconque des revendications 1 à 3 ou un polymère du composé.

**5.** Un matériau optique non linéaire comprenant le composé de l'une quelconque des revendications 1 à 3 ou un polymère formé par polymérisation du composé, en tant qu'au moins un composant constituant.

**6.** Un matériau électro-optique comprenant le composé des revendications 1 à 3 ou un polymère formé par polymérisation du composé, en tant qu'au moins un composant constituant.

**7.** Un composé représenté par la formule générale suivante (I- A) :

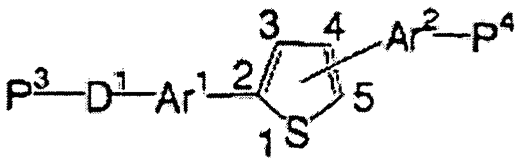

Formule générale (I-A)

dans laquelle :

$Ar^1$ et $Ar^2$ représentent chacun indépendamment une liaison simple, un anneau aromatique ayant de 5 à 14 atomes de carbone, ou un groupe biphényle, à condition qu'au moins l'un de $Ar^1$ et $Ar^2$ représente l'un d'un

anneau naphthalène et d'un groupe biphényle qui peuvent avoir un substituant ;
- $Ar^2$ - $P^4$ se substitue sur la position 4 ou la position 5 d'un anneau thiophène ;
$P^3$ représente un groupe alcoyle linéaire, ramifié, ou cyclique ayant de 1 à 20 atomes de carbone dans lequel un ou plus des - $CH_2$ - dans le groupe alcoyle peut être remplacé par - O -, - CO -, - $NR^1$ - dans lequel $R^1$ représente un groupe alcoyle linéaire, ramifié, ou cyclique ayant de 1 à 20 atomes de carbone, - CH = CH -, ou - C ≡ C -, à condition que des hétéroatomes ne soient pas situés de manière adjacente les uns aux autres, et le groupe alcoyle peut avoir un substituant constitué d'atomes sélectionnés parmi C, H, O, N, S, et un atome d'halogène ;
$P^4$ représente - $P^{4'}$ - $P^6$

dans lequel :

$P^6$ représente un groupe alcoyle linéaire, ramifié, ou cyclique ayant de 1 à 20 atomes de carbone dans lequel un ou plus des - $CH_2$ - dans le groupe alcoyle peut être remplacé par - O -, - CO -, - CH = CH -, ou - C ≡ C -, à condition que des hétéroatomes ne soient pas situés de manière adjacente les uns aux autres, et le groupe alcoyle peut avoir un substituant constitué d'atomes sélectionnés parmi C, H, O, N, S, et un atome d'halogène ; et
$P^{4'}$ représente un groupe attracteur d'électron bivalent sélectionné parmi le groupe constitué d'un groupe ester (- COO -), d'un groupe sulfonyle (- $SO_2$ -), et d'un groupe sulfoxyde (- $SO_3$ -) ;
$D^1$ représente l'un sélectionné parmi le groupe constitué d'un atome d'oxygène (- O -), d'un atome de soufre (- S -), et - $NP^5$ - dans lequel $P^5$ représente un atome d'hydrogène ou un groupe alcoyle linéaire, ramifié, ou cyclique possédant de 1 à 20 atomes de carbone, dans lequel un ou plus des - $CH_2$ - dans le groupe alcoyle peut être remplacé par - O -, - CO -, - CH = CH -, ou - C ≡ C -, à condition que des hétéroatomes ne soient pas situés de manière adjacente les uns aux autres, et le groupe alcoyle peut avoir un substituant constitué d'atomes sélectionnés parmi C, H, O, N, S, et un atome d'halogène, et $P^5$ peut être indépendant de $P^{3,}$ ou lié avec $P^3$ pour former un anneau ; et
l'anneau thiophène peut avoir un substituant.

**8.** Le composé de la revendication 7, dans lequel :

$Ar^1$ est un groupe 1,4-phénylène et $Ar^2$ est un groupe 2,6-naphtalène ; ou
$Ar^1$ est un groupe 2,6-naphtalène et $Ar^2$ est un groupe 1,4-phénylène.

**9.** Le composé de la revendication 7 ou 8, dans lequel $P^3$ et $P^5$ représentent chacun indépendamment un groupe alcoyle linéaire, ramifié, ou cyclique ayant de 1 à 20 atomes de carbone.

**10.** Un élément optique comprenant le composé de l'une quelconque des revendications 7 à 9 ou un polymère du composé.

**11.** Un matériau optique non linéaire comprenant le composé de l'une quelconque des revendications 7 à 9, ou un polymère formé par polymérisation du composé, en tant qu'au moins un composant constituant.

**12.** Un matériau électro-optique comprenant le composé de l'une quelconque des revendications 7 à 9, ou un polymère formé par polymérisation du composé, en tant qu'au moins un composant constituant.

FIG. 1

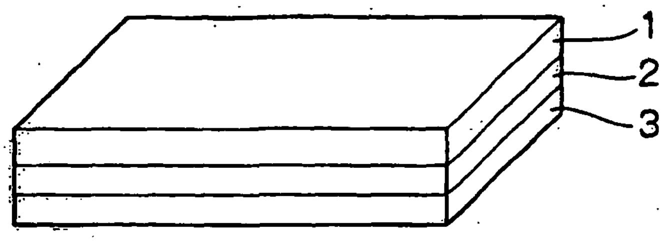


FIG. 2

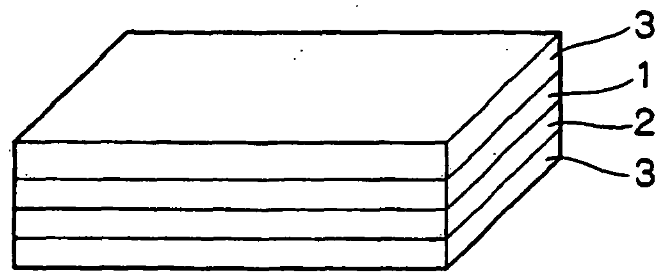

FIG. 3

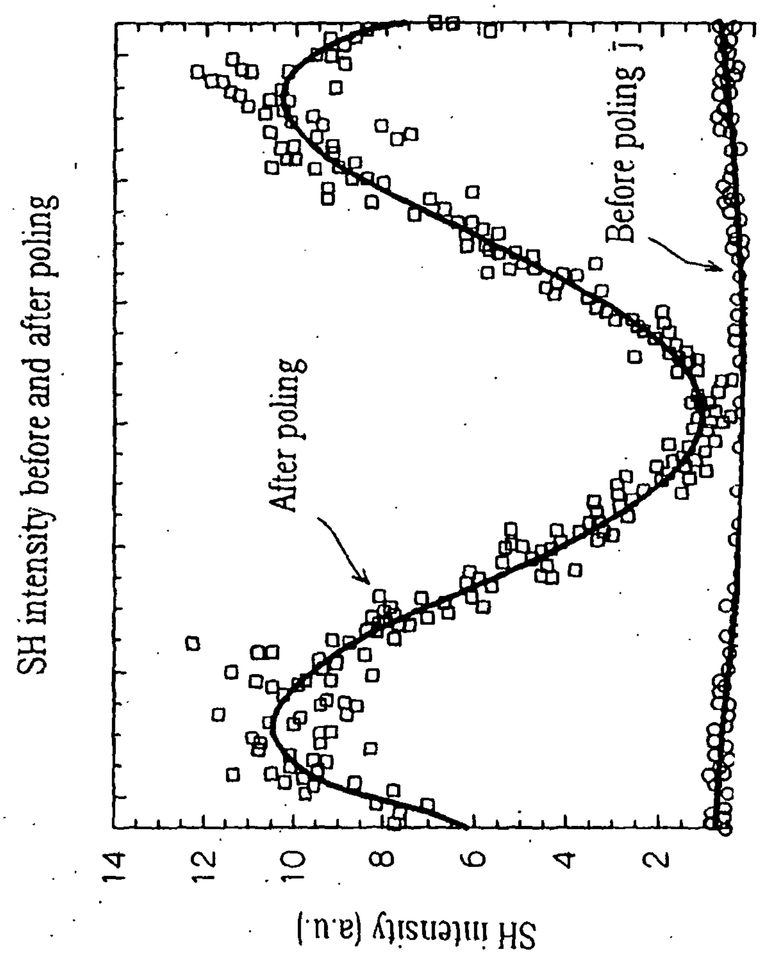
SH intensity before and after poling
After poling
Before poling
SH intensity (a.u.)
14
12
10
8
6
4
2

FIG. 4

Comparison of second harmonic (SH) intensity
(5 μm cell, 150 V (voltage) applied)